(19) Europäisches Patentamt
European Patent Office
Office européen des brevets

(11) EP 2 698 142 B1

(12) # EUROPÄISCHE PATENTSCHRIFT

(45) Veröffentlichungstag und Bekanntmachung des Hinweises auf die Patenterteilung:
**01.02.2017 Patentblatt 2017/05**

(51) Int Cl.:
***A61K 6/083*** $^{(2006.01)}$ ***A61K 6/00*** $^{(2006.01)}$

(21) Anmeldenummer: **13172642.4**

(22) Anmeldetag: **19.06.2013**

(54) **Zahnfüllungsmaterialien und Zahnlacke zur Hemmung der Biofilmbildung von Streptococcus mutans und deren Herstellung**

Dental filling materials and dental varnish for the inhibition of biofilm formation of streptococcus mutans, and their preparation

Matériaux de obturation dentaire et laque dentaire destinés à empêcher la formation de biofilm de Streptococcus mutans et leur fabrication

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.08.2012 DE 102012214540**

(43) Veröffentlichungstag der Anmeldung:
**19.02.2014 Patentblatt 2014/08**

(73) Patentinhaber:
- **VOCO GmbH
27472 Cuxhaven (DE)**
- **Helmholtz-Zentrum für Infektionsforschung GmbH
38124 Braunschweig (DE)**

(72) Erfinder:
- **Wagner-Döbler, Irene
38173 Evessen (DE)**
- **Barg, Andree
21762 Otterndorf (DE)**

(74) Vertreter: **Eisenführ Speiser
Patentanwälte Rechtsanwälte PartGmbB
Postfach 10 60 78
28060 Bremen (DE)**

(56) Entgegenhaltungen:
**DE-A1-102009 035 970 US-A1- 2011 034 708**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Zahnfüllungsmaterialien und Zahnlacke zur Hemmung der Biofilmbildung von *Streptococcus mutans,* enthaltend (a) eine Menge einer Wirksubstanz zur Hemmung der Biofilmbildung, (b) ein Material zur Ausbildung einer Struktur zur Aufnahme und verzögerten Freisetzung der Wirksubstanz und (c) ein Füllstoffsystem. Ein weiterer Aspekt dieser Erfindung betrifft ein Verfahren zum Herstellen von erfindungsgemäßen Zahnfüllungsmaterialien oder Zahnlacken zur Hemmung der Biofilmbildung von *Streptococcus mutans.* Die vorliegende Erfindung betrifft auch ein Kit, umfassend erfindungsgemäße Zahnfüllungsmaterialien oder Zahnlacke und ein oder mehrere weitere Bestandteile. Beschrieben werden auch Verfahren zur Hemmung der Biofilmbildung von *Streptococcus mutans,* Verfahren zur Herstellung eines die Biofilmbildung hemmenden dentalen Bauteils sowie die Verwendung der im nachfolgenden näher spezifizierten Wirksubstanz zur Herstellung eines Dentalmaterials.

Die Erfindung wird durch die unabhängigen Patentansprüche definiert.
Weitere Aspekte der vorliegenden Erfindung ergeben sich aus der nachfolgenden Beschreibung sowie insbesondere aus den beigefügten Patentansprüchen.

Die unter Menschen am weitesten verbreitete Infektionskrankheit Karies entsteht in Form kariöser Läsionen, wenn Biofilmbakterien (Plaquebakterien), primär *Streptococcus mutans* und *Lactobacillaceae,* durch einen anaeroben Stoffwechselmechanismus vergärbarer Kohlenhydrate organische Säuren bilden. Die Säuren diffundieren in die Zahnhartsubstanz und zerstören die kristallinen Phasen des Schmelzes und des Dentins. Durch die Auflösung dieser Mineralien werden strukturelle Veränderungen im Zahnhartgewebe verursacht. Diese Demineralisation findet ab einem pH-Wert von weniger als 5,5 statt. Darüber hinaus kommt es zu einer fortwährenden Ausfällung bestimmter Ionen, wie Kalzium- und Phosphationen, aus dem Speichel. Die Gegenwart dieser Ionen im Mundraum führt zu einer Remineralisation. Ist Fluorid zugegen, wird die Remineralisation zusätzlich gefördert, indem sich säureresistenter Fluorhydroxylapatit bildet. Im dynamischen Milieu der Mundhöhle wechseln sich Demineralisation und Remineralisation ab. Besteht ein Gleichgewicht zwischen diesen Vorgängen, entsteht keine Karies. Wird das Gleichgewicht jedoch gestört, wird durch zunehmenden Verlust an anorganischen Bestandteilen des Schmelzes die Bildung einer kariösen Läsion gefördert.

Dieses Gleichgewicht zwischen Demineralisation und Remineralisation wird durch Biofilme gestört, die sich auf der Grenzfläche zwischen der Zahnoberfläche (dazu gehören auch künstliche Oberflächen, wie Füllungen oder Zahnersatz) und dem restlichen Mundraum bilden. Biofilme bestehen aus einer dünnen Schleimschicht bzw. einem dünnen Schleimfilm, in dem Mikroorganismen (z. B. Bakterien wie *Streptococcus mutans*) eingebettet sind. Neben den Mikroorganismen enthalten Biofilme hauptsächlich Wasser und die von den Mikroorganismen ausgeschiedenen extrazellulären polymeren Substanzen, wie beispielsweise Polysaccharide (z. B. Dextrane, Alginate oder Zellulose), Proteine, Lipide oder Nukleinsäuren, die Hydrogele ausbilden, sodass eine schleimige Matrix entsteht, in der zusätzlich Nährstoffe und andere Substanzen gelöst sein können.

Neben dem Effekt, dass Biofilme das Gleichgewicht zwischen Demineralisation und Remineralisation stören, wird die Demineralisation der Zahnhartsubstanz durch organische Säuren noch verstärkt, die durch einen anaeroben Stoffwechsel von vergärbaren Kohlenhydraten der Mikroorganismen im Biofilm entstehen. In der Zahnheilkunde ist die Bekämpfung von Biofilmen ein wichtiger Aspekt, der immer mehr an Bedeutung gewinnt. Die Bekämpfung von Biofilmen durch Antibiotika-Gabe weist zwei grundlegende Probleme auf. Zum einen bilden Bakterien eine Resistenz gegen Antibiotika, die es den Bakterien erlaubt, die Wirkung von antibiotisch aktiven Substanzen abzuschwächen oder ganz zu neutralisieren, zum anderen ist die Applikation der Antibiotika problematisch. Die orale Gabe von Antibiotika führt nicht zu einer ausreichend hohen Antibiotikakonzentration im Zahnschmelz, während der Gebrauch von antibiotischen Mundspülungen nur Bakterien in der äußeren Biofilmschicht bekämpft.

Es besteht somit ein ständiger Bedarf an Maßnahmen und Mitteln, die die Bildung von Biofilmen im Mundraum hemmen und Biofilme dort bekämpfen wo sie entstehen, insbesondere auf der Zahnoberfläche (dazu gehören auch künstliche Oberflächen, wie Füllungen oder Zahnersatz).

Die Hemmung bzw. die Vermeidung der Biofilmbildung kann nicht nur durch den Einsatz von antibiotischen Mitteln, sondern auch durch andere Maßnahmen erreicht werden. So ist z. B. die Verwendung von proteinabweisenden oder hydrophob beschichteten Oberflächen der Dentalwerkstoffe möglich. Durch solche Maßnahmen wird das Wachstum bzw. die Anhaftung der Bakterien auf den dentalen Werkstoffen erschwert.

Die Verwendung von quartären Ammoniumsalzen als antimikrobielle Additive ist seit langem bekannt. So wird z. B. ein Silan mit quartären Ammoniumgruppen als funktionelle Gruppe von der Firma Microshield hergestellt und zur antibakteriellen Ausrüstung von Filtern, Textilien und Wundauflagen vermarktet. In GB 1 433 303 A werden Füllpartikel für Kunststoffe beschrieben und offenbart, dass diese silanisiert und mit quartären Ammoniumsalzen beschichtet werden. So behandelte Diatomeenerde oder pyrogene Kieselsäuren werden beispielsweise zur Verwendung in Holzbeschichtungen, Dichtungsmassen, Kathetern oder Textilfasern vorgeschlagen.

Gemäß DE 10 2009 035 970 offenbart JP 10025218 A anorganische Füllstoffe, die mit einer antimikrobielle Gruppen enthaltenden Polymerschicht überzogen sind. Die Schicht entsteht durch Polymerisation entsprechender (Meth)acrylat-Monomere, die Phosphonium- oder quartäre Ammoniumgruppen tragen.

Gemäß DE 10 2005 042 078 A1 werden dentale Füllkörper mit antimikrobiell wirksamen Polysacchariden überzogen. Derartige mit einem Polysaccharid beschichtete Füllkörper werden mit einem weiteren Polymer umhüllt (Seite 4, Absatz 0034). In das Polysaccharid Chitosan wird noch eine CC-Doppelbindung eingeführt, um eine Einpolymerisation in das Polymer zu ermöglichen (Seite 4, Absatz 0037). Das Triclosan wird entsprechend auf der Füllkörperoberfläche kovalent gebunden (Seite 5, Absatz 0047).

DE 10 2009 035 970 A1 beschreibt Dentalmaterialien, die mindestens einen auf organische Partikel oder organische Polymerperlen aufgebrachten, antimikrobiellen Wirkstoff enthalten, der nicht kovalent an die Partikel oder Polymerperlen gebunden ist. Bei dem antimikrobiellen Wirkstoff handelt es sich um ein Iminopyridinium-Derivat, Octenidin-Salz, Dequalinium-Salz, Sanguinarin, Akacid®, Chlorhexidin, Alexidin, Hexetidin, Cetylpyridiniumchlorid, Benzalkoniumchlorid, Octenidindihydrochlorid oder Triclosan.

In US 2012/0095114 A1 werden Triclosanderivate an Monomere und Oligomere kovalent gebunden und anschließend zur Bildung von Substraten eingesetzt, die auf dentale Produkte aufgetragen werden können. US 6,207,139 beschreibt verschiedene dentale Produkte, die Triclosan enthalten und eine Wirkung gegen Zahnstein aufweisen.

In US 2012/0171129 A1 werden Benzimidazol-Derivate verwendet, um Biofilme zu entfernen, zu inhibieren oder um der Bildung von Biofilmen vorzubeugen. Die Verwendung der beschriebenen Benzimidazol-Derivate u.a. in Zahnseide, Zahnpasta oder Kaugummi wird ebenfalls beschrieben.

In WO 2004/078154 werden Zahnpflegezusammensetzungen beschrieben, die Partikel enthalten. Diese Partikel hemmen bzw. verhindern die Bildung von Bakterien auf oralen Flächen und auf Zusammensetzungen, die diese Partikel enthalten.

Es war eine primäre Aufgabe der vorliegenden Erfindung, ein Zahnfüllungsmaterial oder einen Zahnlack zur Hemmung der Biofilmbildung von *Streptococcus mutans* anzugeben, die eine Biofilmhemmung erreichen, die besser ist, als die eines ansonsten identisch zusammengesetzten Zahnfüllungsmaterials bzw. Zahnlacks umfassend eine der im Dentalbereich als antimikrobielle Wirkstoffe verwendeten Verbindungen Chlorhexidin und Triclosan.

Diese Aufgabe wird gelöst durch Zahnfüllungsmaterialien oder Zahnlacke zur Hemmung der Biofilmbildung von *Streptococcus mutans* umfassend:

(a) als Wirksubstanz zur Hemmung der Biofilmbildung von *Streptococcus mutans* eine Menge an Carolacton der Formel (II) und

(b) ein Material zur Ausbildung einer Struktur zur Aufnahme und verzögerten Freisetzung der Wirksubstanz, bestehend aus einem, zwei, drei oder mehr polymerisierbaren Monomeren
und

(c) einen oder mehrere partikuläre Füllstoffe
und

(d) ein oder mehrere Additive ausgewählt aus der Gruppe bestehend aus Initiatoren, Beschleuniger und Inhibitoren.

Die Nummerierung der Kohlenstoffatome bezieht sich auf die in der obigen Formel eingezeichneten Nummern.

Zahnfüllungsmaterialien oder Zahnlacke - Einkomponentige und zweikomponentige Systeme:

In der dentalen Praxis werden Zahnfüllungsmaterialien oder Zahnlacke unter anderem nach der Art ihrer Aushärtung unterschieden, die durch Licht- und/oder Autopolymerisation (Lichthärtung und/oder chemische Härtung) erfolgen kann. Lichthärtbare Zahnfüllungsmaterialien oder Zahnlacke, die in Form von Einkomponentenmaterialien vorliegen, sind im Vergleich zu Zahnfüllungsmaterialien oder Zahnlacken, die als Komponente eines zweikomponentigen Systems vorliegen, üblicherweise weniger anfällig für Verarbeitungsfehler, da Einkomponentenmaterialien nicht vor Gebrauch frisch angemischt werden und es somit auch keine Blasenbildung während des Anmischens geben kann, wie sie bei zweikomponentigen Systemen häufig auftritt. Lichthärtbare einkomponentige Zahnfüllungsmaterialien oder Zahnlacke sind somit häufig bevorzugt. Neben einkomponentigen lichthärtbaren Zahnfüllungsmaterialien oder Zahnlacken und zweikomponentigen autopolymerisierbaren (chemisch härtbar, nicht lichthärtbar) Zahnfüllungsmaterialien oder Zahnlacken sind auch sogenannte dualhärtende, zweikomponentige Systeme bekannt, welche durch Lichthärtung und chemische Härtung ausgehärtet werden können.

Erfindungsgemäße Zahnfüllungsmaterialien oder Zahnlacke sind vorzugsweise lichthärtende Einkomponenten-Systeme, in Einzelfällen ist jedoch eine Ausgestaltung bevorzugt, bei der erfindungsgemäße Zahnfüllungsmaterialien oder Zahnlacke als Komponente eines Zweikomponenten-Systems ausgestaltet ist, wobei das Zweikomponenten-System ein chemisch härtendes (nicht lichthärtendes) System oder ein dualhärtendes System ist. Nach dem Anmischen eines zweikomponentigen Systems liegt eine Mischung dieser Komponenten vor, wobei die Mischung ein erfindungsgemäßes Zahnfüllungsmaterial oder ein erfindungsgemäßer Zahnlack ist.

Komponente (a) Wirksubstanz:

Erfindungsgemäße Zahnfüllungsmaterialien oder Zahnlacke umfassen Carolacton der Formel (II)

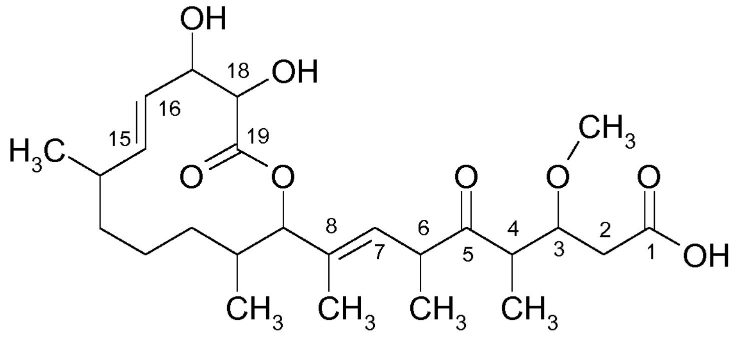

(II).

Die Verwendung von Carolacton als Biofilmhemmer wird bereits in WO 2009/030773A10 beschrieben. Offenbart werden insbesondere pharmazeutische Kompositionen, die die Biofilmbildung hemmen, wobei die Biofilme beispielsweise auf Zahnoberflächen in Form von dentaler Plaque entstehen. Erfindungsgemäße Zahnfüllungsmaterialien oder Zahnlacke werden jedoch nicht beschrieben, insbesondere werden keine Mischungen offenbart, welche eine der Komponenten (b) oder (c) eines erfindungsgemäßen Zahnfüllungsmaterials oder Zahnlacks umfassen. Die WO 2009/030773A10 offenbart auch keine Zusammensetzungen oder Mischungen, die als Zahnfüllungsmaterial oder Zahnlack geeignet sind.

Die Herstellung von Carolacton der Formel (II) ist in der WO 2009/030773A10 offenbart, vgl. dort insbesondere Example 1.

In eigenen Untersuchungen zur antimikrobiellen Wirkung von Triclosan, Chlorhexidin und Carolacton gegenüber *Streptococcus mutans* hat sich für Lösungen dieser Wirkstoffe gezeigt, dass Triclosan und Chlorhexidin auch bei bestimmten geringen Konzentrationen eine sehr starke antimikrobielle Wirkung aufweisen, während Carolacton bei gleicher Konzentration lediglich eine schwache antimikrobielle Wirkung aufweist (siehe hierzu Beispiel 8). Die antimikrobielle Wirkung von Carolacton gegenüber *Streptococcus mutans* ist somit schwächer als die antimikrobielle Wirkung der im Dentalbereich als antimikrobielle Wirkstoffe verwendeten Verbindungen Chlorhexidin und Triclosan. Daher ist es besonders überraschend, dass bei Einsatz von erfindungsgemäßen Zahnfüllungsmaterialien oder Zahnlacken mit Carolacton die Biofilmhemmung wesentlich besser ist, als bei Einsatz entsprechender Zahnfüllungsmaterialien oder Zahnlacke, die anstelle von Carolacton Chlorhexidin bzw. Triclosan enthalten (siehe hierzu Beispiel 9).

Komponente (b) - Material zur Ausbildung einer Struktur zur Aufnahme und verzögerten Freisetzung der Wirksubstanz

Die erfindungsgemäßen Zahnfüllungsmaterialien oder Zahnlacke zur Hemmung der Biofilmbildung von *Streptococcus mutans* umfassen als Komponente

(b) ein Material zur Ausbildung einer Struktur zur Aufnahme und verzögerten Freisetzung der Wirksubstanz, bestehend aus einem, zwei, drei oder mehr polymerisierbaren Monomeren.

Die polymerisierbaren Monomere umfassen dabei vorzugsweise mindestens eine ethylenische Gruppe, wie beispielsweise die in der Dentalchemie üblicherweise in Kompositmaterialien eingesetzten (Meth)acrylate und (Meth)acrylamide.

Bevorzugt sind erfindungsgemäße Zahnfüllungsmaterialien oder Zahnlacke, bei denen das eine polymerisierbare Monomer bzw. eines, zwei, drei, mehrere oder sämtliche der mehreren polymerisierbaren Monomere der Komponente (b) ausgewählt ist bzw. sind aus der Gruppe der (Meth)acrylate und (Meth)acrylamide, vorzugsweise ausgewählt ist bzw. sind aus der Gruppe bestehend aus Ethylenglykoldimethacrylat (EGDMA), 1,6-Hexandioldimethacrylat (HEDMA), Butandioldimethacrylat, Tetraethylenglykoldimethacrylat, Neopentylglykoldimethacrylat, 2-Hydroxypropyl-1,3-dimethacrylat, 3-Hydroxypropyl-1,2-dimethacrylat, Pentaerythritoldimethacrylat, Triethylenglykoldimethacrylat (TEGDMA), Urethandimethacrylat (7,7,9-Trimethyl-4,13-dioxo-3,14-dioxa-5,12-diazahexadecan-1,16-dioxydimethacrylat (UDMA)), Glycerindimethacrylat (GlyDMA), Bisphenol-A-Glycidyl-Methacrylat (Bis-GMA), 1,12-Dodecandioldimethacrylat (DODMA), Hydroxyethylmethacrylat (HEMA), Polyethylenglykoldimethacrylat (PEGDMA) und ethoxyliertes Bisphenol-A-dimethacrylat (EtoBG).

Wie bereits weiter oben beschrieben, werden in der dentalen Praxis Zahnfüllungsmaterialien oder Zahnlacke

durch Licht- und/oder Autopolymerisation (Lichthärtung und/oder chemische Härtung) ausgehärtet. Im Rahmen der vorliegenden Erfindung sind erfindungsgemäße Zahnfüllungsmaterialien oder Zahnlacke besonders bevorzugt, bei denen das eine polymerisierbare Monomer bzw. eines, zwei, drei, mehrere oder sämtliche der mehreren polymerisierbaren Monomere radikalisch oder nicht-radikalisch polymerisierbar sind, vorzugsweise durch lichtinduzierte Reaktion und/oder katalysierte Reaktion radikalisch oder nicht-radikalisch polymerisierbar sind. Bevorzugt sind in der Praxis häufig Zahnfüllungsmaterialien oder Zahnlacke, bei denen das eine polymerisierbare Monomer bzw. eines, zwei, drei, mehrere oder sämtliche der mehreren polymerisierbaren Monomere radikalisch durch lichtinduzierte Reaktion polymerisierbar sind. Solche bevorzugten Zahnfüllungsmaterialien oder Zahnlacke sind vorzugsweise lichthärtende Einkomponenten-Systeme, in Einzelfällen ist jedoch eine Ausgestaltung bevorzugt, bei der erfindungsgemäße Zahnfüllungsmaterialien oder Zahnlacke als Komponente eines Zweikomponenten-Systems ausgestaltet sind, wobei das Zweikomponenten-System ein dualhärtendes System ist.

In der Patentliteratur werden als polymerisierbare Monomere häufig Diester der Acryl- oder Methacrylsäure genannt, (beispielsweise auch in der DE 39 41 629 A1, welche im Wege der Verweisung Bestandteil der vorliegenden Anmeldung ist, insbesondere die Offenbarung im Bereich von Spalte 6, Zeile 15 bis Spalte 8, Zeile 10); diese eignen sich besonders zum Einsatz als polymerisierbares Monomer in erfindungsgemäßen Zahnfüllungsmaterialien oder Zahnlacken.

Bevorzugte polymerisierbare Monomere sind Hydroxylverbindungen, die mindestens eine ethylenische Doppelbindung umfassen. Dabei können alle in der Dentalchemie üblicherweise eingesetzten Hydroxylverbindungen von Acrylaten oder Methacrylaten eingesetzt werden. Bevorzugt sind Hydroxylverbindungen von Methacrylaten, dabei wiederum bevorzugt 2-Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat und 2,2-bis[4-[3-methacryloyloxy-2-hydroxypropoxy]phenyl]propan.

Die genannten polymerisierbaren Monomere können einzeln oder in Gemischen eingesetzt werden.

Komponente (c) - partikuläre Füllstoffe

Die erfindungsgemäßen Zahnfüllungsmaterialien oder Zahnlacke zur Hemmung der Biofilmbildung von *Streptococcus mutans* umfassen als Komponente (c) einen oder mehrere (chemisch unterschiedliche) partikuläre Füllstoffe.

Unter partikulären Füllstoffen werden Füllstoffe verstanden, die in Form von Teilchen vorliegen. Die Partikel (Teilchen) können dabei eine beliebige Gestalt besitzen; der Begriff "partikulärer Füllstoff" umfasst somit insbesondere formunabhängig Feststoffe, Fasern, Röhren, auch (in englischer Sprache) "doughnuts" (also Tori), "(multi)dimples" "ringwülstige", kompakte und poröse, isolierte und aggregierte/agglomerierte Feststoffe.

Abhängig vom Herstellungsverfahren der partikulären Füllstoffe können die Partikel teilweise agglomeriert oder aggregiert vorliegen.

Die partikulären Füllstoffe können dabei chemisch einheitlich sein (d. h. sämtlich die gleiche chemische Zusammensetzung besitzen) oder als Mischung chemisch unterschiedlicher Füllstoffe eingesetzt werden. Zur Optimierung der Produkteigenschaften können die partikulären Füllstoffe in unterschiedlichen Korngrößen in die Rezepturen eingebracht werden. Die Füllstoffe bzw. die Mischung mehrerer chemisch unterschiedlicher Füllstoffe können eine unimodale oder polymodale, beispielsweise eine bimodale Verteilung, aufweisen.

Geeignete anorganische partikuläre Füllstoffe sind beispielsweise amorphe Materialien auf der Basis von Mischoxiden aus $SiO_2$, $ZrO_2$ und/oder $TiO_2$, mikrofeine Füllstoffe wie pyrogene und/oder nicht-agglomerierte und/oder nicht-aggregierte Kieselsäure oder Fällungskieselsäure sowie Makro- oder Mini-Füllstoffe wie Quarz-Dentalglas oder Glaspulver, Bariumsilikatgläser, Bariumfluorsilikatgläser, Strontiumsilikatgläser, Strontiumborsilikat, Li/Al-Silikatgläser, Bariumgläser, Calciumsilikate, Natriumaluminiumsilikate, Fluoraluminiumsilikatgläser, Oxide von Aluminium oder Silicium, Zeolithe, Apatit, Zirkonsilikate, schwerlösliche Metallsalze wie Bariumsulfat oder Calciumfluorid sowie röntgenopake Füllstoffe wie Ytterbiumfluorid.

Zum besseren Einbau in die aus Komponente (b) resultierende Struktur zur Aufnahme und verzögerten Freisetzung der Wirksubstanz (die Polymermatrix) können die partikulären Füllstoffe oberflächenmodifiziert sein. Die organische Oberflächenbehandlung der Füllstoffe verbessert üblicherweise die Kompatibilität der Partikeloberfläche mit der organischen Bindemittelphase, da die an sich hydrophile Teilchenoberfläche durch die Oberflächenbehandlung hydrophobiert wird und sich dann besser mit der Kunststoffmatrix verträgt. Die Oberflächenmodifizierung wird bevorzugt so durchgeführt, dass die anorganische Oberfläche bei Aushärtung des Materials durch Copolymerisation kovalent in das entstehende Polymer eingebunden wird. Häufig wird die anorganische Oberfläche silanisiert. Hierbei werden vorhydrolysierte Methacryloxyalkyltrialkoxysilane, wie beispielsweise 3-Methacryloxypropyltrimethoxysilan, eingesetzt, bei denen die bei der Hydrolyse gebildeten Silanolgruppen mit den freien Hydroxylgruppen der Füllstoffoberfläche reagieren. Angaben über Modifizierungsreagentien und Ausführungsarten sind u.a. aus DE 24 05 578 A1, US 2002/0065337, DE 195 08 586 A1, WO 00/69392 sowie US 6,387,981 bekannt.

Mit Sol-Gel-Verfahren ist die Herstellung nanoskaliger Metall- und Schwermetalloxide sowie von Mischoxiden möglich.

Gemäß einem bevorzugten Verfahren zur Herstellung von kolloidalem Kieselsol wird von Wasserglas ausgegangen. Hierbei wird eine wässrige Lösung von Natriumsilikat mittels eines Ionenaustauschers entionisiert, wodurch Kieselsäure gebildet wird. Diese Säure ist instabil und polymerisiert zu kleinen Partikeln, aus denen dann die nanoskaligen Kieselsäuren gebildet werden. Durch ein geeignetes Einstellen der Prozessparameter können auch hier enge Partikelgrößenverteilungen erhalten werden.

Wie flammhydrolytisch hergestellte, pyrogene Kieselsäuren, die kommerziell in unterschiedlichen Partikelgrößen durch die Produktserien "Aerosile" (Degussa) oder "CAB-O-SIL" (Cabot Corporation) erhältlich sind, können auch die nach alternativen Verfahren hergestellten Kieselsäuren in unterschiedlichen Größen durch die Produktserien "Highlink" (Clariant), "Nalco" (Nalco Chemical Company), "Nanocryl" (Nanoresins), "Bidzil" (Eka Chemicals), "Levasil" (Fa. H.C. Starck), "NexSil" (Nyacol) oder "Ludox" (du Pont) kommerziell erstanden werden.

Zur Einstellung der Rheologie können erfindungsgemäße Zahnfüllungsmaterialien oder Zahnlacke unterschiedliche Kieselsäuren, bevorzugt pyrogene und/oder nicht-agglomerierte und/oder nicht-aggregierte Kieselsäuren, enthalten.

Besonders bevorzugt sind erfindungsgemäße Zahnfüllungsmaterialien oder Zahnlacke, umfassend in oder als Komponente (c) einen oder mehrere anorganische partikuläre Füllstoffe, wobei der eine bzw. einer oder mehrere oder sämtliche der anorganischen partikulären Füllstoffe vorzugsweise ausgewählt sind aus der Gruppe bestehend aus silanisierten $SiO_2$-Nanopartikeln, pyrogenen und/oder nicht-agglomerierten und/oder nicht-aggregierten Kieselsäuren, nicht-aggregierten, nicht-agglomerierten $SiO_2$-Nanopartikeln, Dentalglaspartikeln, silanisierten Dentalglaspartikeln, Zirkoniumoxidpartikeln, Aluminiumoxidpartikeln und mittels organischer Strukturelemente oberflächenmodifizierten, anorganischen Füllstoffpartikeln.

Komponente (d)- Initiatoren, Beschleuniger und Inhibitoren

Erfindungsgemäße Zahnfüllungsmaterialien oder Zahnlacke umfassen ein oder mehrere Additive ausgewählt aus der Gruppe bestehend aus Initiatoren, Beschleuniger und Inhibitoren, d.h. Additive, welche (i) eine Lichthärtung und/oder (ii) chemische Härtung des einen oder der mehreren polymerisierbaren Monomere ermöglichen oder unterstützen. Weitere Additive können zusätzlich zu diesen Additiven eingesetzt werden.

Beispiele für Additive, welche eine Lichthärtung des einen oder der mehreren polymerisierbaren Monomere ermöglichen oder unterstützen, sind Katalysatoren, die nur photosensibilisierend wirken (Photosensibilisatoren, Photoinitiatoren), sowie Beschleuniger (Akzeleratoren, Co-Initiatoren), die vorzugsweise in Kombination mit Photosensibilisatoren eingesetzt werden.

Beispiele für geeignete Photosensibilisatoren sind alpha-Diketone (z.B. Campherchinon), Benzoinalkylether, Thioxanthone, Benzophenone, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1 oder in der DE 39 41 629 C2, welche im Wege der Verweisung Bestandteil der vorliegenden Anmeldung sind.

Beispiele für geeignete Beschleuniger, die zusammen mit den Photosensibilisatoren eingesetzt werden, sind tertiäre Amine (z.B. Ethyl-*p-N,N*-dimethylaminobenzoat (DABE)), sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2, welche im Wege der Verweisung Bestandteil der vorliegenden Anmeldung sind.

Weitere geeignete Additive (Initiatoren sowie Initiatorkombinationen) sind in der DE 601 16 142 beschrieben, welche im Wege der Verweisung Bestandteil der vorliegenden Anmeldung sind.

Die im Rahmen der vorliegenden Erfindung verwendbaren Photoinitiatoren sind vorzugsweise dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Co-initiatoren, die Aushärtung von erfindungsgemäßen Zahnfüllungsmaterialien oder Zahnlacken bewirken können.

Das Absorptionsmaximum von Campherchinon (CC) liegt bei ca. 470 nm und somit im Bereich des blauen Lichts. Campherchinon (CC) zählt zu den $PI_2$-Initiatoren und wird regelmäßig zusammen mit einem Coinitiator eingesetzt.

Vorzugsweise enthalten erfindungsgemäße Zahnfüllungsmaterialien oder Zahnlacke die Kombination eines alpha-Diketons und eines aromatischen tertiären Amins, bevorzugt ist die Kombination von Campherchinon (CC) und Ethyl-*p-N,N*-dimethylaminobenzoat (DABE).

Ebenfalls bevorzugt ist die weitere Kombination des Systems "alpha-Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyl-diphenylphosphinoxid. Bezüglich der Strukturen geeigneter Phosphinoxide zum Einsatz in einer erfindungsgemäßen Zusammensetzung wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600 29 481 T2, EP 0 980 682 B1, EP 0 948 955 B1, EP 1 236 459 B1 und EP 0 173 567 A2 verwiesen, welche im Wege der Verweisung Bestandteil der vorliegenden Anmeldung sind.

Die in diesen Druckschriften angegebenen Phosphinoxide eignen sich besonders allein oder in Kombination mit dem System "alpha-Diketon/Amin" als Photopolymerisationsinitiatorsystem in den erfindungsgemäßen Zahnfüllungsmaterialien oder Zahnlacken.

Alternativ können auch Boratsalze, wie sie beispielsweise in US 4,772,530, US 4,954,414, US 4,874,450, US 5,055,372 und US 5,057,393 beschrieben sind, als Photoinitiatoren Verwendung finden.

Weitere im Rahmen der vorliegenden Erfindung geeignete Photoinitiatoren sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer Science and Technology, Vol. II, Elsevier Applied Science, London, New York 1993 beschrieben, welche im Wege der Verweisung Bestandteil der vorliegenden Anmeldung sind.

Dem Fachmann sind diverse Initiatoren für eine chemische Aushärtung bekannt, die auch im Rahmen der vorliegenden Erfindung eingesetzt werden können. Es sei insoweit exemplarisch auf die EP 1 720 506 verwiesen.

Bevorzugte Initiatoren zur chemischen Härtung von erfindungsgemäßen Zahnfüllungsmaterialien oder Zahnlacken sind Benzoylperoxid, Lauroylperoxid und Dibenzoylperoxid. Die besagten chemischen Initiatoren, insbesondere Dibenzoylperoxid, werden vorzugsweise in Kombination mit Aminen eingesetzt, wie z.B. N,N-Dimethyl-p-toluidin, N,N-Dihydroxyethyl-p-toluidin sowie strukturverwandte Amine.

Die Peroxide und die Amine werden dabei üblicherweise auf zwei unterschiedliche Komponenten eines Dentalmaterials aufgeteilt, wobei vorzugsweise beide Komponenten sowie die nach Mischung der Komponenten vorliegende Mischung erfindungsgemäße Zahnfüllungsmaterialien oder Zahnlacke sind. Beim Mischen der aminhaltigen Komponente (sogenannte Basispaste) mit der peroxidhaltigen Komponente (sogenannte Initiator- oder Katalysatorpaste) wird durch die Reaktion von Amin und Peroxid (Redoxreaktion) die radikalische Reaktion initiiert.

Dualhärtende Systeme sind sowohl chemisch härtbare als auch lichthärtbare Systeme, die üblicherweise zwei Komponenten umfassen, wobei vorzugsweise beide Komponenten sowie die nach Mischung der Komponenten vorliegende Mischung erfindungsgemäße Zahnfüllungsmaterialien oder Zahnlacke sind. Dualhärtende Systeme umfassen eine Kombination aus Photoinitiatoren und Initiatoren zur chemischen Aushärtung.

Beispielsweise kann die Basispaste eines dualhärtenden Systems ein erfindungsgemäßes Zahnfüllungsmaterial oder ein erfindungsgemäßer Zahnlack sein und neben einer aminhaltigen Komponente zusätzlich einen Photoinitiator enthalten, so dass die Basispaste entweder allein als lichthärtender oder zusammen mit der Initiatorpaste als licht- und chemisch härtender Dentalwerkstoff eingesetzt werden kann.

Alternativ oder zusätzlich zu den Peroxid/Aminsystemen können im Rahmen der vorliegenden Erfindung in chemisch härtenden Systemen Redoxsysteme eingesetzt werden, die Barbitursäuren bzw. Barbitursäurederivate und/oder Malonylsulfamide umfassen.

Von den Barbitursäuresystemen sind die sogenannten "Bredereck-Systeme" von hoher Bedeutung. Beispiele geeigneter "Bredereck-Systeme" sowie Verweise auf die entsprechende Patentliteratur findet man in der EP 1 839 640 sowie in DE 14 95 520, WO 02/092021 oder in WO 02/092023.

Geeignete Malonylsulfamide sind in der EP 0 059 451. Bevorzugte Verbindungen sind dabei 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Diocytyl-4-isobutylmalonylsulfamid.

Ferner können Schwefelverbindungen in der Oxidationsstufe +2 oder +4 wie Natriumbenzolsulfinat oder Natriumparatoluolsulfinat eingesetzt werden.

Zur Beschleunigung der Aushärtung kann die Polymerisation in Gegenwart von Schwermetallverbindungen wie Ce, Fe, Cu, Mn, Co, Sn oder Zn durchgeführt werden, wobei Kupferverbindungen besonders bevorzugt sind. Die Schwermetallverbindungen werden bevorzugt in Form löslicher organischer Verbindungen eingesetzt. Bevorzugte Kupferverbindungen sind dabei Kupferbenzoat, Kupferacetat, Kupferethylhexanoat, Kupferdi(methacrylat), Kupferacetylacetonat und Kupfernaphthenat.

Die erfindungsgemäßen Zahnfüllungsmaterialien oder Zahnlacke enthalten vorzugsweise einen oder mehrere Inhibitoren, auch Stabilisatoren genannt. Diese werden einer Mischung zugesetzt, um eine Spontanpolymerisation zu vermeiden. Sie reagieren mit vorzeitig entstehenden Radikalen, die abgefangen werden, verhindern eine vorzeitige Polymerisation und erhöhen die Lagerstabilität der lichthärtbaren Zusammensetzung. Gängige Inhibitoren sind Phenolderivate wie Hydrochinonmonomethylether (HQME) oder 2,6-Di-tert.butyl-4-methylphenol (BHT). Weitere Inhibitoren wie 2,2 Diphenyl-1-picrylhydrazyl-, Galvinoxyl-, Triphenylmethyl-Radikale, 2,3,6,6,-Tetramethylpiperidinyl-1-oxylradikale (TEMPO) sowie Derivate von TEMPO oder Phenothiazin sowie Derivate dieser Verbindung werden in der EP 0 783 880 B1 beschrieben, welche im Wege der Verweisung Bestandteil der vorliegenden Anmeldung sind. Alternative Inhibitoren sind in der DE 101 19 831 A1 oder in der EP 1 563 821 A1 angegeben, welche im Wege der Verweisung

Bestandteil der vorliegenden Anmeldung sind.

Komponente (e) - metallische Additive

Vorzugsweise umfasst ein erfindungsgemäßes Zahnfüllungsmaterial bzw. ein erfindungsgemäßer Zahnlack ein oder mehrere Additive ausgewählt aus der Gruppe bestehend aus Silber, Silberionen freisetzende Substanzen, Kupfer, Kupferionen freisetzende Substanzen, Zink und Zinkionen freisetzende Substanzen. Diese Additive können zusätzlich zu anderen Additiven eingesetzt werden und werden üblicherweise eingesetzt, um die antimikrobielle Wirkung der erfindungsgemäßen Zahnfüllungsmaterialien oder Zahnlacke noch weiter zu verbessern und die Biofilmbildung noch besser zu hemmen.

Komponente (f) - Weichmacher

Im Rahmen des vorliegenden Textes werden unter Weichmachern nicht weiter polymerisierbaren Polymere verstanden, die nicht an einer radikalischen Polymerisation mit (Meth)acrylaten teilnehmen und zum Zwecke einer sogenannten "äußeren Weichmachung" eingesetzt werden können. Beispielsweise handelt es sich bei Polyethylenglycol (PEG) um einen Weichmacher im Sinne dieser Definition.

In eigenen Untersuchungen hat sich gezeigt, dass in manchen Fällen die Anwesenheit von Weichmachern (wie vorstehend definiert) in erfindungsgemäßen Zahnfüllungsmaterialien oder Zahnlacken einen positiven Einfluss auf die gesteuerte Freisetzung der Wirksubstanz der Komponente (a) hat. Insbesondere sind dabei Weichmacher bevorzugt, die eine hohe Hydrophilie aufweisen und den Wirkstoff daher besonders kontrolliert aufnehmen und gesteuert abgeben können.

Besonders bevorzugt sind daher erfindungsgemäße Zahnfüllungsmaterialien oder Zahnlacke umfassend als Komponente (f) ein, zwei, drei oder mehr Weichmacher, vorzugsweise Polyethylenglycol als Weichmacher.

Besonders bevorzugt sind daher erfindungsgemäße Zahnfüllungsmaterialien oder Zahnlacke, wobei insgesamt 0 Gew.-% bis 25 Gew.-%, bevorzugt 0,01 Gew.-% bis 15 Gew.-%, besonders bevorzugt 0,1 Gew.-% bis 5 Gew.-% eines Weichmachers, bezogen auf das Gesamtgewicht des Zahnfüllungsmaterials bzw. Zahnlacks, enthalten sind.

Komponente (g) - sonstige Bestandteile

Vorzugsweise umfassen erfindungsgemäße Zahnfüllungsmaterialien oder Zahnlacke ein oder mehrere Additive, bei denen es sich nicht um Substanzen handelt, die bereits als Bestandteil einer der vorstehend diskutierten Komponenten aufgefasst werden können. Solche Bestandteile werden als "sonstige Bestandteile" bezeichnet.

So können z. B. UV-Absorber, die beispielsweise durch ihre konjugierten Doppelbindungssyteme und aromatischen Ringe befähigt sind, UV-Strahlung zu absorbieren, Bestandteil eines erfindungsgemäßen Zahnfüllungsmaterials oder Zahnlacks sein. Beispiele für UV-Absorber sind 2-Hydroxy-4-methoxybenzophenon, Salicylsäurephenylester oder 3-(2'-Hydroxy-5'-methylphenyl)-benzotriazol.

Da die Zähne möglichst naturgetreu wiederherzustellen sind, ist es notwendig, die Zahnfüllungsmaterialien oder Zahnlacke in den unterschiedlichsten Farbtönen bereitzustellen. Man benutzt zu diesem Zweck in der Regel anorganische Farbstoffe sowie organische Pigmente in sehr geringen Mengen. Solche Farbstoffe bzw. Pigmente sind ebenfalls sonstige Bestandteile, welche als oder in Komponente (g) in einer erfindungsgemäßen Zahnfüllungsmaterialien oder Zahnlacke vorliegen können.

Neben den oben aufgeführten sonstigen Bestandteilen sind dem Fachmann weitere sonstige Bestandteile bekannt, die in üblichen Zahnfüllungsmaterialien oder Zahnlacken verwendet werden. Weitere Bestandteile werden häufig in geringen Mengen als Additive zugegeben, um z. B. die mechanischen oder optischen Eigenschaften von Zahnfüllungsmaterialien oder Zahnlacken bzw. von ausgehärteten Zahnfüllungsmaterialien oder Zahnlacken zu verbessern.

Erfindungsgemäß bevorzugt sind Zahnfüllungsmaterialien oder Zahnlacke, umfassend

(e) ein oder mehrere Additive ausgewählt aus der Gruppe bestehend aus Silber, Silberionen freisetzende Substanzen, Kupfer, Kupferionen freisetzende Substanzen, Zink und Zinkionen freisetzende Substanzen
und/oder

(f) ein, zwei, drei oder mehr Weichmacher, vorzugsweise Polyethylenglycol als Weichmacher
und/oder

(g) ein oder mehrere sonstige Bestandteile.

Bestandteile, die technisch und begrifflich zwei oder mehr Komponenten zugeordnet werden können, werden insbesondere für die Zwecke quantitativer Angaben zur Vermeidung von Missverständnissen jeweils der früher genannten Komponente zugeordnet (also (a) vor (b) vor (c) vor (d) vor (e) vor (f) vor (g)).

Der Fachmann wird in erfindungsgemäßen Zahnfüllungsmaterialien oder Zahnlacken den Anteil an Carolacton der Formel (II) so wählen, dass der von ihm gewünschte Effekt zur Hemmung der Biofilmbildung von *Streptococcus mutans* erreicht wird, wobei er vorzugsweise Sorge trägt, einerseits keine zu großen Gesamtmengen an Carolacton der Formel (II) einzusetzen und andererseits nicht lediglich eine so geringe Menge an Carolacton der Formel (II) vorzusehen, dass eine Hemmung der Biofilmbildung von *Streptococcus mutans* nicht beziehungsweise nicht in signifikantem Maße zu spüren ist.

Erfindungsgemäß bevorzugt sind Zahnfüllungsmaterialien oder Zahnlacke, umfassend als Wirksubstanz zur Hemmung der Biofilmbildung von *Streptococcus mutans* insgesamt 0,001 μg/mL bis 2500 μg/mL, bevorzugt 0,01 μg/mL bis 250 μg/mL, besonders bevorzugt 0,1 μg/mL bis 25 μg/mL an Carolacton der Formel (II) bezogen auf das Volumen des Zahnfüllungsmaterials oder Zahnlacks.

In einer besonders bevorzugten Ausführungsform sind erfindungsgemäße Zahnfüllungs-materialien oder Zahnlacke dadurch gekennzeichnet, dass Carolacton der Formel (II) in solch einer Menge in dem Zahnfüllungsmaterial oder Zahnlack enthalten ist, dass die Biofilmhemmung auf (a) den Zahnfüllungsmaterialien oder Zahnlacken und/oder auf (b) den durch Härtung ausgehärteten Zahnfüllungsmaterialien oder Zahnlacken mindestens 10%, bevorzugt mindestens 30%, besonders bevorzugt mindestens 50% beträgt.

Besonders bevorzugte erfindungsgemäße Zahnfüllungsmaterialien bestehen aus

- als Komponente (a)
  insgesamt 0,001 μg/mL bis 2500 μg/mL, bevorzugt 0,01 μg/mL bis 250 μg/mL, besonders bevorzugt 0,1 μg/mL bis 25 μg/mL Carolacton der Formel (II), bezogen auf das Volumen des Zahnfüllungsmaterials,

- als Komponente (b)
  insgesamt 5 Gew.-% bis 50 Gew.-%, bevorzugt 10 Gew.-% bis 35 Gew.-%, besonders bevorzugt 15 Gew.-% bis 25 Gew.-% des Materials zur Ausbildung einer Struktur zur Aufnahme und verzögerten Freisetzung der Wirksubstanz bestehend aus ein, zwei, drei oder mehr polymerisierbaren Monomeren, bezogen auf das Gesamtgewicht des Zahnfüllungsmaterials,

- als Komponente (c)
  insgesamt 40 Gew.-% bis 90 Gew.-%, bevorzugt 55 Gew.-% bis 85 Gew.-%, besonders bevorzugt 70 Gew.-% bis 80 Gew.-% des anorganischen Füllstoffs oder des Füllstoffgemisches, bezogen auf das Gesamtgewicht des Zahnfüllungsmaterials,

- als Komponente (d)
  insgesamt 0,001 Gew.-% bis 10 Gew.-%, bevorzugt 0,01 Gew.-% bis 5 Gew.-%, besonders bevorzugt 0,01 Gew.-% bis 2 Gew.-% der Initiatoren, Beschleuniger und/oder Inhibitoren, bezogen auf das Gesamtgewicht des Zahnfüllungsmaterials,

- als Komponente (f)
  insgesamt 0 Gew.-% bis 25 Gew.-%, bevorzugt 0,01 Gew.-% bis 15 Gew.-%, besonders bevorzugt 0,1 Gew.-% bis 5 Gew.-% eines Weichmachers, bezogen auf das Gesamtgewicht des Zahnfüllungsmaterials, und

- als Komponente (g)
  insgesamt 0 Gew.-% bis 15 Gew.-%, weitere Bestandteile bezogen auf das Gesamtgewicht des Zahnfüllungsmaterials.

Besonders bevorzugte erfindungsgemäße Zahnlacke bestehen aus

- als Komponente (a)
  insgesamt 0,001 μg/mL bis 2500 μg/mL, bevorzugt 0,01 μg/mL bis 250 μg/mL, besonders bevorzugt 0,1 μg/mL bis 25 μg/mL Carolacton der Formel (II), bezogen auf das Volumen des Zahnlacks,

- als Komponente (b)
  insgesamt 40 Gew.-% bis 99 Gew.-%, bevorzugt 50 Gew.-% bis 97 Gew.-%, besonders bevorzugt 60 Gew.-% bis 95 Gew.-% des Materials zur Ausbildung einer Struktur zur Aufnahme und verzögerten Freisetzung der Wirksubstanz bestehend aus ein, zwei, drei oder mehr polymerisierbaren Monomeren, bezogen auf das Gesamtgewicht des

Zahnlacks,

- als Komponente (c)
insgesamt 0,1 Gew.-% bis 10 Gew.-%, bevorzugt 0,5 Gew.-% bis 8 Gew.-%, besonders bevorzugt 1 Gew.-% bis 5 Gew.-% des anorganischen Füllstoffs oder des Füllstoffgemisches, bezogen auf das Gesamtgewicht des Zahnlacks,

- als Komponente (d)
insgesamt 0,001 Gew.-% bis 10 Gew.-%, bevorzugt 0,01 Gew.-% bis 5 Gew.-%, besonders bevorzugt 0,01 Gew.-% bis 2 Gew.-% der Initiatoren, Beschleuniger und/oder Inhibitoren, bezogen auf das Gesamtgewicht des Zahnlacks,

- als Komponente (f)
insgesamt 0 Gew.-% bis 15 Gew.-%, bevorzugt 0,01 Gew.-% bis 10 Gew.-%, besonders bevorzugt 0,1 Gew.-% bis 5 Gew.-% des Weichmachers bezogen auf das Gesamtgewicht des Zahnlacks, und

- als Komponente (g)
insgesamt 0 Gew.-% bis 15 Gew.-%, weitere Bestandteile bezogen auf das Gesamtgewicht des Zahnfüllungsmaterials.

Damit möglichst gute mechanischen Eigenschaften (beispielsweise Biegefestigkeit oder Abrasionsresistenz) der ausgehärteten erfindungsgemäßen Zahnfüllungsmaterialien oder Zahnlacke erreicht werden können und gleichzeitig eine gute Aufnahme und verzögerte Freisetzung der Wirksubstanz erfolgen kann, hat es sich in eigenen Untersuchungen gezeigt, dass es vorteilhaft ist, die einzelnen Komponenten sehr gut miteinander zu vermischen, so dass das Resultat eine Mischung ist, die eine homogene Phase umfasst, in der Füllstoffpartikel gleichmäßig (homogen) verteilt vorliegen. Die homogene Phase bildet somit die kontinuierliche Phase einer Dispersion. Erfindungsgemäß besonders bevorzugt sind dementsprechend Zahnfüllungsmaterialien oder Zahnlacke, wobei die Komponente (a) mit der Komponente (b) eine homogene Phase bildet und/oder wobei Komponente (c) gleichmäßig in der Mischung von (a) und (b) suspendiert ist.

Erfindungsgemäß bevorzugt sind Zahnfüllungsmaterialien oder Zahnlacke zur Anwendung in einem Verfahren zur therapeutischen (einschließlich prophylaktischen) Behandlung des menschlichen oder tierischen Körpers,

vorzugsweise zur Anwendung

- in einem Verfahren zum Inhibieren, Reduzieren oder Verhindern von Zahnschäden, insbesondere von Zahnschäden, die durch *Streptococcus mutans* hervorgerufen werden,
und/oder

- in einem Verfahren zum Inhibieren, Reduzieren oder Verhindern von Caries dentium.

Erfindungsgemäß besonders bevorzugt sind Zahnfüllungsmaterialien oder Zahnlacke zur Anwendung in einem therapeutischen Verfahren zum Inhibieren, Reduzieren oder Verhindern von Biofilmen, vorzugsweise von bakteriellen Biofilmen.

In einer bevorzugten Ausführungsform betrifft die Erfindung Zahnfüllungsmaterialien zur Hemmung der Biofilmbildung von *Streptococcus mutans,* vorzugsweise zur Anwendung in einem Verfahren zur therapeutischen (einschließlich prophylaktischen) Behandlung des menschlichen oder tierischen Körpers, umfassend:

(a) als Wirksubstanz zur Hemmung der Biofilmbildung von *Streptococcus mutans* insgesamt 0,1 μg/mL bis 25 μg/mL einer Verbindungen der Formel (II), bezogen auf das Gesamtgewicht des Zahnfüllungsmaterials,

(b) insgesamt 15 Gew.-% bis 25 Gew.-% eines Materials zur Ausbildung einer Struktur zur Aufnahme und verzögerten Freisetzung der Wirksubstanz bestehend aus ein, zwei, drei oder mehr polymerisierbaren Monomeren, bezogen auf das Gesamtgewicht des Zahnfüllungsmaterials,
wobei das eine polymerisierbare Monomer bzw. eines, zwei, drei, mehrere oder sämtliche der mehreren polymerisierbaren Monomere ausgewählt ist bzw. sind aus der Gruppe der (Meth)acrylate, vorzugsweise ausgewählt ist bzw. sind aus der Gruppe bestehend aus Triethylenglykoldimethacrylat (TEGDMA), Urethandimethacrylat (7,7,9-Trimethyl-4,13-dioxo-3,14-dioxa-5,12-diazahexadecan-1,16-dioxydimethacrylat (UDMA)), Glycerindimethacrylat (GlyDMA), Bisphenol-A-Glycidyl-Methacrylat (Bis-GMA), 1,12-Dodecandioldimethacrylat (DODMA), Hydroxyethylmethacrylat (HEMA), Polyethylenglykoldimethacrylat (PEGDMA) und ethoxyliertes Bisphenol-A-dimethacrylat

(EtoBG),

(c) insgesamt 70 Gew.-% bis 80 Gew.-% eines oder mehrerer anorganischer partikulärer Füllstoffe, wobei der eine bzw. einer oder mehrere oder sämtliche der anorganischen Füllstoffe ausgewählt sind aus der Gruppe bestehend aus silanisierten Dentalglaspartikeln und pyrogenen und/oder nicht-agglomerierten und/oder nicht-aggregierten Kieselsäuren, bezogen auf das Gesamtgewicht des Zahnfüllungsmaterials,
und

(d) insgesamt 0,01 Gew.-% bis 2 Gew.-% eines oder mehrerer Additive ausgewählt aus der Gruppe bestehend aus Initiatoren, Beschleuniger und Inhibitoren, bezogen auf das Gesamtgewicht des Zahnfüllungsmaterials
sowie optional

(e) ein oder mehrere Additive ausgewählt aus der Gruppe bestehend aus Silber, Silberionen freisetzende Substanzen, Kupfer, Kupferionen freisetzende Substanzen, Zink und Zinkionen freisetzende Substanzen sowie optional

(f) Polyethylenglycol als Weichmacher
sowie optional

(g) ein oder mehrere sonstige Bestandteile.

In einer bevorzugten Ausführungsform betrifft die Erfindung Zahnlacke zur Hemmung der Biofilmbildung von *Streptococcus mutans,* vorzugsweise zur Anwendung in einem Verfahren zur therapeutischen (einschließlich prophylaktischen) Behandlung des menschlichen oder tierischen Körpers, umfassend:

(a) als Wirksubstanz zur Hemmung der Biofilmbildung von *Streptococcus mutans* insgesamt 0,1 μg/mL bis 25 μg/mL einer Verbindungen der Formel (II), bezogen auf das Gesamtgewicht des Zahnlacks,
(b) insgesamt 60 Gew.-% bis 90 Gew.-% eines Materials zur Ausbildung einer Struktur zur Aufnahme und verzögerten Freisetzung der Wirksubstanz bestehend aus ein, zwei, drei oder mehr polymerisierbaren Monomeren, bezogen auf das Gesamtgewicht des Zahnlacks,
wobei das eine polymerisierbare Monomer bzw. eines, zwei, drei, mehrere oder sämtliche der mehreren polymerisierbaren Monomere ausgewählt ist bzw. sind aus der Gruppe der (Meth)acrylate, vorzugsweise ausgewählt ist bzw. sind aus der Gruppe bestehend aus Triethylenglykoldimethacrylat (TEGDMA), Urethandimethacrylat (7,7,9-Trimethyl-4,13-dioxo-3,14-dioxa-5,12-diazahexadecan-1,16-dioxydimethacrylat (UDMA)), Glycerindimethacrylat (GlyDMA), Bisphenol-A-Glycidyl-Methacrylat (Bis-GMA), 1,12-Dodecandioldimethacrylat (DODMA), Hydroxyethylmethacrylat (HEMA), Polyethylenglykoldimethacrylat (PEGDMA) und ethoxyliertes Bisphenol-A-dimethacrylat (EtoBG),
(c) insgesamt 1 Gew.-% bis 5 Gew.-% eines oder mehrerer anorganischer partikulärer Füllstoffe, wobei der eine bzw. einer oder mehrere oder sämtliche der anorganischen Füllstoffe ausgewählt sind aus der Gruppe bestehend aus pyrogenen und/oder nicht-agglomerierten und/oder nicht-aggregierten Kieselsäuren, bezogen auf das Gesamtgewicht des Zahnlacks,
und
(d) insgesamt 0,01 Gew.-% bis 2 Gew.-% eines oder mehrerer Additive ausgewählt aus der Gruppe bestehend aus Initiatoren, Beschleuniger und Inhibitoren, bezogen auf das Gesamtgewicht des Zahnlacks,
sowie optional
(e) ein oder mehrere Additive ausgewählt aus der Gruppe bestehend aus Silber, Silberionen freisetzende Substanzen, Kupfer, Kupferionen freisetzende Substanzen, Zink und Zinkionen freisetzende Substanzen
sowie optional
(f) Polyethylenglycol als Weichmacher
sowie optional
(g) ein oder mehrere sonstige Bestandteile.

Die Erfindung betrifft zudem ein Verfahren zum Herstellen von erfindungsgemäßen Zahnfüllungsmaterialien oder Zahnlacken, mit den folgenden Schritten:

(i) Bereitstellen oder Herstellen (a) einer Menge an Carolacton der Formel (II), als Wirksubstanz zur Hemmung der Biofilmbildung von *Streptococcus mutans,* vorzugsweise als alkoholische Lösung,
(ii) Bereitstellen oder Herstellen (b) eines Materials zur Ausbildung einer Struktur zur Aufnahme und verzögerten Freisetzung der Wirksubstanz bestehend aus ein, zwei, drei oder mehr polymerisierbaren Monomeren,

(iii) Bereitstellen oder Herstellen (c) eines oder mehrerer partikulärer Füllstoffe
(iv) Bereitstellen oder Herstellen (d) eines oder mehrerer Additive ausgewählt aus der Gruppe bestehend aus Initiatoren, Beschleuniger und Inhibitoren,
(v) optional Bereitstellen oder Herstellen

(e) eines oder mehrerer Additive ausgewählt aus der Gruppe bestehend aus Silber, Silberionen freisetzende Substanzen, Kupfer, Kupferionen freisetzende Substanzen, Zink und Zinkionen freisetzende Substanzen,

(f) von ein, zwei, drei oder mehr Weichmachern, vorzugsweise von Polyethylenglycol als Weichmacher und/oder

(g) von ein oder mehr sonstigen Bestandteilen

und
(vi) Vermischen der bereitgestellten und/oder hergestellten Komponenten.

Die Erfindung betrifft auch ein Kit umfassend:

- ein erfindungsgemäßes Zahnfüllungsmaterial oder einen erfindungsgemäßen Zahnlack (wie oben definiert, vorzugsweise wie oben als bevorzugt bezeichnet) und

- ein oder mehrere weitere Bestandteile ausgewählt aus der Gruppe bestehend aus Haftvermittler, Abutments, Implantate, Formwerkzeuge, Abformmaterialien, Farbskalen, Farbmustern und Lichthärtegeräten.

Ein weiterer Aspekt der vorliegenden Erfindung ist die Verwendung von Carolacton der Formel (II) zur Herstellung eines erfindungsgemäßen Zahnfüllungsmaterials oder Zahnlacks.
Die Erfindung betrifft auch ein (therapeutisches, insbesondere prophylaktisches, oder kosmetisches) Verfahren zum Hemmen der Biofilmbildung von *Streptococcus mutans* in der Mundhöhle eines Patienten mit folgenden Schritten:

- Bereitstellen oder Herstellen eines erfindungsgemäßen Zahnfüllungsmaterials oder Zahnlacks (wie oben definiert, vorzugsweise wie oben als bevorzugt bezeichnet),

- Applizieren des bereitgestellten bzw. hergestellten Zahnfüllungsmaterials oder Zahnlacks auf eine Fläche in der Mundhöhle eines Patienten, vorzugsweise auf einer Zahnfläche in der Mundhöhle eines Patienten,

- optional Formen des applizierten Zahnfüllungsmaterials oder Zahnlacks auf der Fläche in der Mundhöhle des Patienten,

- Aushärten oder härten lassen der applizierten, optional des geformten Zahnfüllungsmaterials oder Zahnlacks auf der Fläche in der Mundhöhle des Patienten und

- optional Nachbehandeln des ausgehärteten Zahnfüllungsmaterials oder Zahnlacks auf der Fläche in der Mundhöhle des Patienten, vorzugsweise durch Polieren und/oder Schleifen.

Die Erfindung betrifft auch ein (erstes) Verfahren zur Herstellung eines die Biofilmbildung hemmenden dentalen Bauteils, mit folgenden Schritten:

- Bereitstellen eines dentalen Bauteils,

- Bereitstellen oder Herstellen eines Zahnfüllungsmaterials oder Zahnlacks (wie oben definiert, vorzugsweise wie oben als bevorzugt bezeichnet),

- Applizieren des bereitgestellten bzw. hergestellten Zahnfüllungsmaterials oder Zahnlacks auf das dentale Bauteil,

- optional Formen des applizierten Zahnfüllungsmaterials oder Zahnlacks auf dem dentalen Bauteil,

- optional Aushärten oder härten lassen des applizierten, optional des geformten Zahnfüllungsmaterials oder Zahnlacks auf dem dentalen Bauteil und

- optional Nachbehandeln des ausgehärteten Zahnfüllungsmaterials oder Zahnlacks auf dem dentalen Bauteil, vorzugsweise durch Polieren und/oder Schleifen.

Als Alternative zum ersten Verfahren zur Herstellung eines die Biofilmbildung hemmenden dentalen Bauteils betrifft die Erfindung auch ein (zweites) Verfahren zur Herstellung eines die Biofilmbildung hemmenden dentalen Bauteils, mit folgenden Schritten:

- Bereitstellen oder Herstellen eines erfindungsgemäßen Zahnfüllungsmaterials (wie oben definiert, vorzugsweise wie oben als bevorzugt bezeichnet),
- Formen des Zahnfüllungsmaterials,
- Aushärten oder härten lassen des geformten Zahnfüllungsmaterials und
- optional Nachbehandeln des ausgehärteten geformten Zahnfüllungsmaterials.

Beide Verfahren zur Herstellung eines die Biofilmbildung hemmenden dentalen Bauteils können bevorzugt als sogenanntes chair-side-Verfahren ausgestaltet werden. Es versteht sich jedoch, dass die erfindungsgemäßen Verfahren zwar als chair-side-Verfahren ausgestaltet sein können, jedoch nicht als chair-side-Vefahren ausgebildet sein müssen. In einer Vielzahl von Fällen ist es vorteilhaft, anstelle eines chair-side-Verfahrens eine Verfahrensgestaltung zu wählen, bei der kein einzelner Verfahrensschritt im Mund des oder am Patienten erfolgt. Derartige Verfahrensgestaltungen werden auch als lab-side-Verfahren bezeichnet.
Weitere Aspekte der vorliegenden Erfindung ergeben sich aus den nachfolgenden Vorschriften, Beispielen und Patentansprüchen.

Herstellungsbeispiel: Herstellung von mittels organischer Strukturelemente oberflächenmodifizierten Kieselsäurepartikeln:

Allgemeine Vorschrift

Es wird eine kolloidale Dispersion von nasschemisch hergestellten, anorganischen Füllstoffpartikeln geeigneter mittlerer Teilchengröße bereitgestellt, wobei als Dispersionsmittel eine organische, wasserlösliche Matrix, beispielsweise Isopropanol, eingesetzt wird.
Die Dispersion wird vermischt mit

(a) einer ausreichenden Menge eines Oberflächenmodifizierungsmittels, welches einerseits Alkoxysilan-Gruppen besitzt und andererseits organische Gruppen, welche mit radikalisch polymerisierbaren Monomeren unter Bildung kovalenter Bindungen umsetzbar sind (ein solches Oberflächenmodifizierungsmittel ist z.B. 3-Methacryloyloxypropyltrialkoxysilan),

(b) der zwei bis zehnfachen molaren Menge Wasser, bezogen auf das Oberflächenmodifizierungsmittel, sowie

(c) ca. 1 Gew.-%, bezogen auf das Oberflächenmodifizierungsmittel, Methacrylsäure.

Die resultierende Mischung wird bei 50-80°C mindestens 8h gerührt, sodass die Oberflächenmodifizierung der eingesetzten anorganischen Füllstoffpartikel vollständig ablaufen kann.

Herstellung von silanisierten Nano $SiO_2$ Partikeln

Die silanisierten Nano $SiO_2$ Partikel werden gemäß der allgemeinen Vorschrift zur Herstellung von mittels organischer Strukturelemente oberflächenmodifizierten Kieselsäurepartikeln hergestellt, wobei die nachfolgenden Materialien verwendet werden. Diese silanisierten Nano $SiO_2$ Partikel werden in den Beispielen 1, 2, 3, 4 und 7 eingesetzt.
Materialien zur Herstellung von silanisierten Nano $SiO_2$ Partikeln:

864g einer ethanolischen, 20%igen Suspension nicht flammhydrolytisch hergestellter Kieselsäurepartikel (mit einer mittleren Teilchengröße von 40 nm),
40g gamma-Methacryloyloxypropylsilan (23 % bezogen auf den Feststoffanteil, zur Oberflächenmodifizierung),
0,4g Methacrylsäure und

18g Wasser.

Beispiel 1: Herstellen eines erfindungsgemäßen Zahnfüllungsmaterials

Die folgenden Komponenten wurden in ein Becherglas eingewogen, wobei sich die Gewichtsprozentangaben auf die Gesamtmenge der fertigen Mischung beziehen:

| | | |
|---|---|---|
| ethoxyliertes Bisphenol-A-dimethacrylat (EtoBG) | 10,00 Gew.-% | Komponente b) |
| Triethylenglykoldimethacrylat (TEGDMA) | 9,9975 Gew.-% | Komponente b) |
| 1,6-Hexandioldimethacrylat (HEDMA) | 4,75 Gew.-% | Komponente b) |
| Campherchinon | 0,10 Gew.-% | Komponente d) |
| Ethyl-*p*-*N,N*-dimethylaminobenzoat (DABE) | 0,15 Gew.-% | Komponente d) |
| Carolacton | 0,0025 Gew.-% | Komponente a) |

Anschließend wurde das Gemisch mit einem KPG Rührer für 12 Stunden homogenisiert. Zu dieser Mischung wurden die Füllstoffe:

| | | |
|---|---|---|
| Nano $SiO_2$ Partikel, silanisiert | 25,00 Gew.-% | Komponente c) |
| Dentalglas 3,5 $\mu$m | 50,00 Gew.-% | Komponente c) |

hinzugegeben und durch inniges Vermischen mit einem Doppelplaneten-Mischer wurde eine homogene Paste hergestellt und anschließend unter Vakuum entlüftet.

Beispiel 2: Herstellen eines erfindungsgemäßen Zahnfüllungsmaterials

Die folgenden Komponenten wurden in ein Becherglas eingewogen, wobei sich die Gewichtsprozentangaben auf die Gesamtmenge der fertigen Mischung beziehen:

| | | |
|---|---|---|
| ethoxyliertes Bisphenol-A-dimethacrylat (EtoBG) | 8,00 Gew.-% | Komponente b) |
| Triethylenglykoldimethacrylat (TEGDMA) | 7,998 Gew.-% | Komponente b) |
| 1,6-Hexandioldimethacrylat (HEDMA) | 3,8 Gew.-% | Komponente b) |
| Campherchinon | 0,08 Gew.-% | Komponente d) |
| Ethyl-*p*-*N,N*-dimethylaminobenzoat (DABE) | 0,12 Gew.-% | Komponente d) |
| Carolacton | 0,002 Gew.-% | Komponente a) |

Anschließend wurde das Gemisch mit einem KPG Rührer für 12 Stunden homogenisiert. Zu dieser Mischung wurden die Füllstoffe:

| | | |
|---|---|---|
| Nano $SiO_2$ Partikel, silanisiert | 9,00 Gew.-% | Komponente c) |
| Dentalglas silanisiert, d50= 3,5 $\mu$m | 58,00 Gew.-% | Komponente c) |
| Dentalglas silanisiert, d50= 1,0 $\mu$m | 13,00 Gew.-% | Komponente c) |

hinzugegeben und durch inniges Vermischen mit einem Doppelplaneten-Mischer wurde eine homogene Paste hergestellt und anschließend unter Vakuum entlüftet.

Beispiel 3: Herstellen eines erfindungsgemäßen Zahnfüllungsmaterials

Die folgenden Komponenten wurden in ein Becherglas eingewogen, wobei sich die Gewichtsprozentangaben auf die Gesamtmenge der fertigen Mischung beziehen:

| | | |
|---|---|---|
| ethoxyliertes Bisphenol-A-dimethacrylat (EtoBG) | 8,80 Gew.-% | Komponente b) |
| Triethylenglykoldimethacrylat (TEGDMA) | 8,7978 Gew.-% | Komponente b) |
| 1,6-Hexandioldimethacrylat (HEDMA) | 4,12 Gew.-% | Komponente b) |
| Campherchinon | 0,088 Gew.-% | Komponente d) |

(fortgesetzt)

| | | |
|---|---|---|
| Ethyl-*p*-*N,N*-dimethylaminobenzoat (DABE) | 0,132 Gew.-% | Komponente d) |
| Carolacton | 0,0022 Gew.-% | Komponente a) |

Anschließend wurde das Gemisch mit einem KPG Rührer für 12 Stunden homogenisiert. Zu dieser Mischung wurden die Füllstoffe:

| | | |
|---|---|---|
| Nano $SiO_2$ Partikel, silanisiert | 10,80 Gew.-% | Komponente c) |
| Dentalglas silanisiert, d50= 3,5 µm | 53,40 Gew.-% | Komponente c) |
| Dentalglas silanisiert, d50= 1,0 µm | 13,80 Gew.-% | Komponente c) |

hinzugegeben und durch inniges Vermischen mit einem Doppelplaneten-Mischer wurde eine homogene Paste hergestellt und anschließend unter Vakuum entlüftet.

Beispiel 4: Herstellen eines erfindungsgemäßen Zahnfüllungsmaterials

Die folgenden Komponenten wurden in ein Becherglas eingewogen, wobei sich die Gewichtsprozentangaben auf die Gesamtmenge der fertigen Mischung beziehen:

| | | |
|---|---|---|
| Bisphenol-A-Glycidyl-Methacrylat (Bis-GMA) | 7,04 Gew.-% | Komponente b) |
| Triethylenglykoldimethacrylat (TEGDMA) | 7,81 Gew.-% | Komponente b) |
| 7,7,9-Trimethyl-4,13-dioxo-3,14-dioxa-5,12-diazahexadecan-1,16-dioxydimethacrylat (UDMA) | 7,04 Gew.-% | Komponente b) |
| Campherchinon | 0,044 Gew.-% | Komponente d) |
| Ethyl-*p*-*N,N*-dimethylaminobenzoat (DABE) | 0,066 Gew.-% | Komponente d) |
| Carolacton | 0,0025 Gew.-% | Komponente a) |

Anschließend wurde das Gemisch mit einem KPG Rührer für 12 Stunden homogenisiert. Zu dieser Mischung wurden die Füllstoffe:

| | | |
|---|---|---|
| Nano $SiO_2$ Partikel, silanisiert | 10,80 Gew.-% | Komponente c) |
| Dentalglas silanisiert, d50= 3,5 µm | 53,3975 Gew.-% | Komponente c) |
| Dentalglas silanisiert, d50= 1,0 µm | 13,80 Gew.-% | Komponente c) |

hinzugegeben und durch inniges Vermischen mit einem Doppelplaneten-Mischer wurde eine homogene Paste hergestellt und anschließend unter Vakuum entlüftet.

Beispiel 5: Herstellen eines erfindungsgemäßen Zahnlacks

Die folgenden Komponenten wurden in ein Becherglas eingewogen, wobei sich die Gewichtsprozentangaben auf die Gesamtmenge der fertigen Mischung beziehen:

| | | |
|---|---|---|
| Trimellitsäureanhydrid-4-methacryloyloxyethylester (4-META) | 14,9975 Gew.-% | Komponente b) |
| 1,6-Hexandioldimethacrylat (HEDMA) | 7,5 Gew.-% | Komponente b) |
| 7,7,9-Trimethyl-4,13-dioxo-3,14-dioxa-5,12-diazahexadecan-1,16-dioxydimethacrylat (UDMA) | 47 Gew.-% | Komponente b) |
| Triethylenglykoldimethacrylat (TEGDMA) | 10 Gew.-% | Komponente b) |
| 1,12-Dodecandioldimethacrylat (DODMA) | 7,5 Gew.-% | Komponente b) |

(fortgesetzt)

| | | |
|---|---|---|
| Bisphenol-A-Glycidyl-Methacrylat (Bis-GMA) | 6 Gew.-% | Komponente b) |
| PEG 300 | 3,5 Gew.-% | Komponente f) |
| Campherchinon | 0,81 Gew.-% | Komponente d) |
| Ethyl-*p*-*N,N*-dimethylaminobenzoat (DABE) | 1,215 Gew.-% | Komponente d) |
| Carolacton | 0,0025 Gew.-% | Komponente a) |

Anschließend wurde das Gemisch mit einem KPG Rührer für 12 Stunden homogenisiert. Zu dieser Mischung wurden die Füllstoffe:

| | | |
|---|---|---|
| Aerosil R972 | 1,5 Gew.-% | Komponente c) |

hinzugegeben und durch inniges Vermischen mit einem Doppelplaneten-Mischer wurde eine homogene Paste hergestellt und anschließend unter Vakuum entlüftet.

Beispiel 6: Herstellen eines erfindungsgemäßen Zahnlacks

Die folgenden Komponenten wurden in ein Becherglas eingewogen, wobei sich die Gewichtsprozentangaben auf die Gesamtmenge der fertigen Mischung beziehen:

| | | |
|---|---|---|
| 1,6-Hexandioldimethacrylat (HEDMA) | 12,5 Gew.-% | Komponente b) |
| 7,7,9-Trimethyl-4,13-dioxo-3,14-dioxa-5,12-diazahexadecan-1,16-dioxydimethacrylat (UDMA) | 31 Gew.-% | Komponente b) |
| Triethylenglykoldimethacrylat (TEGDMA) | 19,9975 Gew.-% | Komponente b) |
| 1,12-Dodecandioldimethacrylat (DODMA) | 7,5 Gew.-% | Komponente b) |
| Ethoxyliertes Bisphenol-A-dimethacrylat (EtoBG) | 16 Gew.-% | |
| Bisphenol-A-Glycidyl-Methacrylat (Bis-GMA) | 6 Gew.-% | Komponente b) |
| PEG 300 | 3,5 Gew.-% | Komponente f) |
| Campherchinon | 0,81 Gew.-% | Komponente d) |
| Ethyl-*p*-*N,N*-dimethylaminobenzoat (DABE) | 1,215 Gew.-% | Komponente d) |
| Carolacton | 0,0025 Gew.-% | Komponente a) |

Anschließend wurde das Gemisch mit einem KPG Rührer für 12 Stunden homogenisiert. Zu dieser Mischung wurden die Füllstoffe:

| | | |
|---|---|---|
| Aerosil R972 | 1,5 Gew.-% | Komponente c) |

hinzugegeben und durch inniges Vermischen mit einem Doppelplaneten-Mischer wurde eine homogene Paste hergestellt und anschließend unter Vakuum entlüftet.

Beispiel 7: Herstellen eines erfindungsgemäßen Zahnfüllungsmaterials

Beispiel 7a: Herstellung des Katalysator-Gemisches

Für die Herstellung des Katalysator-Gemisches wurden die folgenden Komponenten in ein Becherglas eingewogen, wobei sich die Gewichtsprozentangaben auf die Gesamtmenge des fertigen Katalysator-Gemisches beziehen:

| | | |
|---|---|---|
| 7,7,9-Trimethyl-4,13-dioxo-3,14-dioxa-5,12-diazahexadecan-1,16-dioxydimethacrylat (UDMA) | 12 Gew.-% | Komponente a) |

(fortgesetzt)

| | | |
|---|---|---|
| Polyethylenglykoldimethacrylat | 12,3475 Gew.-% | Komponente a) |
| Stabilisator | 0,15 Gew.-% | Komponente b) |
| Benzoylperoxid | 0,5 Gew.-% | Komponente b) |
| Carolacton | 0,0025 Gew.-% | Komponente a) |

Anschließend wurde das Gemisch mit einem KPG Rührer für 12 Stunden homogenisiert. Zu dieser Mischung wurden die Füllstoffe:

| | | |
|---|---|---|
| Nano $SiO_2$ Partikel, silanisiert | 25,00 Gew.-% | Komponente c) |
| Dentalglas 3,5 µm | 50,00 Gew.-% | Komponente c) |

hinzugegeben und durch inniges Vermischen mit einem Doppelplaneten-Mischer wurde eine homogene Paste hergestellt und anschließend unter Vakuum entlüftet.

Beispiel 7b: Herstellung des Basis-Gemisches

Für die Herstellung des Basis-Gemisches wurden die folgenden Komponenten in ein Becherglas eingewogen, wobei sich die Gewichtsprozentangaben auf die Gesamtmenge des fertigen Katalysator-Gemisches beziehen:

| | | |
|---|---|---|
| 7,7,9-Trimethyl-4,13-dioxo-3,14-dioxa-5,12-diazahexadecan-1,16-dioxydimethacrylat (UDMA) | 12,1975 Gew.-% | Komponente a) |
| Polyethylenglykoldimethacrylat | 12,00 Gew.-% | Komponente a) |
| *N,N*-Bis(2-hydroxyethyl)-*p*-toluidin | 0,5 Gew.-% | Komponente b) |
| Campherchinon | 0,15 Gew.-% | Komponente b) |
| Coinitiator | 0,15 Gew.-% | Komponente b) |
| Carolacton | 0,0025 Gew.-% | Komponente a) |

Das Gemisch wurde mit einem KPG Rührer für 12 Stunden homogenisiert. Zu dieser Mischung wurden die Füllstoffe:

| | | |
|---|---|---|
| Nano $SiO_2$ Partikel, silanisiert | 25,00 Gew.-% | Komponente c) |
| Dentalglas 3,5 µm | 50,00 Gew.-% | Komponente c) |

hinzugegeben und durch inniges Vermischen mit einem Doppelplaneten-Mischer wurde eine homogene Paste hergestellt und anschließend unter Vakuum entlüftet.

Beispiel 8: Vergleich der koloniebildenden Einheiten von *Streptococcus mutans* (Colonyforming-Units (CFU)) nach Behandlung mit Carolacton, Chlorhexidin und Triclosan

In einem THB Nährmedium wurde eine Übernachtkultur von *Streptococcus mutans* UA159 anaerob kultiviert. Diese Übernachtkultur wurde bis zu einer optischen Dichte (bei 620 nm; $OD_{620}$) von 0,01 - 0,03 mit THB (mit Zugabe von 0,5% (G/V) Saccharose) verdünnt, sodass eine verdünnte Bakteriensuspension erhalten wurde.

Stammlösungen und entsprechende verdünnte Lösungen von Chlorhexidin, Trichlosan und Carolacton wurden in Methanol hergestellt. 10 µl dieser jeweiligen zu testenden Lösungen wurden in Mikrotiterplatten pipettiert, pro Substanz und Konzentration wurde 9 parallele Wells eingesetzt. Das Methanol wurde unter dem Abzug verdampft, und dann 200 µl der verdünnten Bakteriensuspension zugegeben. Die Endkonzentrationen der Wirkstoffe in den 200 µl Bakteriensuspension waren 0,25 µg/ml und 2,5 µg/ml, Die Platten wurden ohne Schütteln für 20 h anaerob bei 37°C inkubiert. Danach wurde der Überstand abpipettiert, der Biofilm mit physiologischer Kochsalzlösung gewaschen (0.85 % NaCl),

und der Biofilm mit einer Pipette abgekratzt. Die Biofilme aus drei parallelen Wells wurden in 500 µl PBS suspendiert. Von dieser Biofilmsuspension wurde eine serielle Verdünnung in PBS hergestellt. Nach jedem Verdünnungsschritt wurde 1 min gevortext. Von jeder Verdünnung wurden dreimal 50 µl gleichmäßig auf Agarplatten (TH-Medium) verteilt. Die Platten wurden drei Tage im CO2-Schrank bei 37°C inkubiert, anschließend wurden die Kolonie bildenden Einheiten (KBE bzw. cfu - colony forming units) gezählt und die Bakteriendichte bezogen auf 1000 ml Biofilm-Suspension berechnet.

Die erhaltenen Ergebnisse zeigen im Vergleich deutlich, dass Triclosan und Chlorhexidin bei beiden genannten Endkonzentrationen zu einer sehr starken Verminderung der koloniebildenden Einheiten führen, während Carolacton die Zahl der koloniebildenden Einheiten lediglich schwach reduziert. Triclosan und Chlorhexidin weisen somit eine hohe antimikrobielle Wirkung in Lösung auf, während die antimikrobielle Wirkung von Carolacton lediglich schwach ist.

Beispiel 9: Vergleichende Bestimmung der Biofilmhemmung bei Einsatz von Zahnfüllungsmaterialien

Vorbereiten der Mikrotiterplatte:

Als erfindungsgemäßes Zahnfüllungsmaterial wurde die Mischung aus Beispiel 2 verwendet.

Zusätzlich wurden drei Zahnfüllungsmaterialien analog zu Beispiel 2 hergestellt, wobei bei einer ersten Vergleichsmischung Carolacton durch Triclosan ausgetauscht, bei einer zweiten Vergleichsmischung Carolacton durch Chlorhexidin ausgetauscht und bei einer dritten Vergleichsmischung Carolacton weggelassen (Kontrolle) wurde.

Von jedem der hergestellten Zahnfüllungsmaterial wurden durch Aushärten des Zahnfüllungsmaterials je 10 dentale Prüfkörper mit einem Durchmesser von 7 mm und einer Höhe von 1 mm hergestellt und mit einer geringen Mengen der jeweiligen Zahnfüllungsmaterials in insgesamt 40 Näpfchen einer 96-well Mikrotiterplatte aus Polystyrol (Greiner Bio-One, µClear Plate Black, Frickenhausen, Deutschland) geklebt.

Kultivierung des Biofilms

In einem THB Nährmedium wurde eine Übernachtkultur von *Streptococcus mutans* UA159 unter anaeroben Bedingungen hergestellt. Die erhaltenen Übernachtkultur wurde bis zu einer optischen Dichte (bei 620 nm; $OD_{620}$) von 0,01 - 0,03 mit THB (mit Zugabe von 0,5% (G/V) Saccharose) verdünnt. Anschließend wurden je 200 µL der verdünnten Übernachtkultur in die mit den dentalen Prüfkörpern bestückten Näpfchen (Wells) der 96-well Mikrotiterplatte pipettiert. Die Mikrotiterplatte wurde anschließend für 20 Stunden unter anaeroben Bedingungen in einem Brutschrank bei 37°C inkubiert.

Bestimmung der Biofilmhemmung

Die Bestimmung der Biofilmhemmung erfolgte mit einem LIVE/DEAD BacLight Kit L13152 (Invitrogen, Molecular Probes, Inc. Eugene, OR, USA) unter Beachtung der Herstelleranleitung. Das Kit besteht aus zwei Färbelösungen, Propidiumjodit und SYTO9, die jeweils Nukleinsäuren verfärben. Die Überstände der Biofilme in den einzelnen Näpfchen (Wells) der 96-well Mikrotiterplatte wurden entfernt. Um planktonische oder lose gebundene Bakterien zu entfernen, wurde der Biofilm vorsichtig mit 200µL einer 0,85%igen NaCl Lösung gewaschen, mit 200 µL einer 1:1 Mischung aus Propidiumjodit und SYTO9 versetzt und anschließend für 15 Minuten und Raumtemperatur unter Ausschluss von Licht stehen gelassen. Die Messung der Fluoreszenz erfolgte an einem Wallac Victor3™1420Multilabel Counter (Perkin-Elmer Life Sciences) ausgestattet mit Detektoren und Filtern zum Messen der Fluoreszenz bei 630 nm (rot) und 535 nm (grün). Die Biofilmhemmung wird als Quotient zwischen dem Quotienten aus grüner und roter Fluoreszenz der Probe mit Wirkstoff (Carolacton, Chrlorhexidin oder Triclosan) und dem Quotienten aus grüner und roter Fluoreszenz der Probe ohne Wirkstoff (Kontrolle) in Prozent angegeben. Die Ergebnisse sind in der nachfolgenden Tabelle 1 zusammengefasst:

**Tabelle 1**

| Wirkstoff | Biofilmhemmung [%] |
|---|---|
| Carolacton (25 µg/ml) | 51 |
| Triclosan (2500 µg/ml) | 1 |
| Chlorhexidin (2500 µg/ml) | 1 |

Die Ergebnisse zeigen deutlich, dass Carolacton eine sehr gute Biofilmhemmung bewirkt, während Triclosan und Chlorhexidin die Biofilmbildung kaum beeinflussen.

Bestimmung koloniebildender Einheiten im Biofilm:

Die Ergebnisse zur Bestimmung koloniebildender Einheiten im Biofilm (Vorgehensweise bei der Bestimmung wie in Beispiel 8) zeigen überraschenderweise eine starke Hemmwirkung von Carolacton auf den Biofilm, während weder Triclosan noch Chlorhexidin, obwohl eingesetzt in der hundertfachen Konzentration von Carolacton, zu einer starken Verminderung der Zahl von KBE führen.

**Tabelle 2**

| | Mittelwert [cfu/mL] | Standardfehler [cfu/mL] | Verbleibende KBE % (Normierung auf Kontrolle) | Hemmung % |
|---|---|---|---|---|
| Zahnfüllungsmaterial mit Triclosan 2500 μg/ml | 6.593.333 | 1.311.111 | 89 | 11 |
| Zahnfüllungsmaterial mit Chlorhexidin 2500 μg/ml | 4.220.000 | 786.667 | 57 | 43 |
| Zahnfüllungsmaterial mit Carolacton 25 μg/ml | 113.333 | 22.222 | 2 | 98 |
| Zahnfüllungsmaterial ohne Wirkstoff (Kontrolle) | 7.426.667 | 2.111.111 | 100 (= normiert) | 0 |

Beispiel 10: Bestimmung der Biegefestigkeit des gehärteten erfindungsgemäßen Zahnfüllungsmaterials

Die Bestimmung der Biegefestigkeit erfolgt analog zur ISO 4049. Dazu wird ein Prüfkörper mit einer Dimension von 2x2 mm vermessen. Bei lichthärtenden Zusammensetzungen wird für 20 Sekunden bei geeigneter Wellenlänge ausgehärtet.

Für die Bestimmung der Biegefestigkeit nach Temperaturzyklen ("Biegefestigkeit TC"), wird der Prüfkörper 3000 Temperaturzyklen zwischen 5 und 55°C ausgesetzt (hierzu wird er alternierend jeweils für 1 Minute in ein Bad der Temperatur 55 °C gelegt und anschließend für 1 Minute in ein Bad der Temperatur 5 °C) und anschließend vermessen.

Die Bestimmung der Biegefestigkeit erfolgte für die Zahnfüllungsmaterialien aus den Beispielen 2, 3 und 4. Die Ergebnisse sind in der nachfolgenden Tabelle 3 zusammengefasst.

**Tabelle 3**

| | Beispiel 2 | Beispiel 3 | Beispiel 4 |
|---|---|---|---|
| Biegefestigkeit (24 h) [MPa] | 141 | 152 | 121 |
| Biegefestigkeit TC [MPa] | 122 | 127 | 106 |

Beispiel 11: Bestimmung der Wasseraufnahme

Die Wasseraufnahme wurde analog zu ISO 4049 bestimmt. Dazu wurden die Zahnfüllungsmaterialien luftblasenfrei in entsprechende Teflonformen eingefüllt, mit Folien und Glasplatten bedeckt und mit einer Schraubzwinge wurden die Überschüsse herausgepresst. Die Probekörper mit einem Durchmesser von 15,0 ± 0,1 mm und einer Höhe von 1,0 ± 0,1 mm wurden segmentweise lichtgehärtet. Anschließend wurden die Probekörper in einen Exsikkator bei 37°C aufbewahrt. Nach 22 Stunden wurden die Probekörper entnommen, für 2 Stunden in einen zweiten Exsikkator auf 23°C gebracht und dann auf 0,1 mg gewogen. Dieser Zyklus wurde so lange wiederholt, bis eine konstante Masse, $m_1$, erreicht war.

Nach dem vollständigen Trocknen wurde der Durchmesser zweimal rechtwinklig zueinander mit einer Messgenauigkeit von 0,01 mm gemessen und daraus der mittlere Durchmesser berechnet. Die Dicke des Probekörpers wurde in der Mitte und an vier in gleichem Abstand liegenden Stellen des Randes auf 0,01 mm gemessen. Aus dem mittleren Durchmesser und der mittleren Dicke wurde das Volumen, V, berechnet.

Anschließend wurden die Probekörper für 7 Tage in Wasser bei 37°C gelagert. Danach wurden die Probekörper herausgenommen, mit Wasser abgespült und abgetupft, bis auf der Oberfläche keine Feuchtigkeit mehr sichtbar war. Die Probekörper wurden 15 s in der Luft hin- und her geschwenkt und 1 min nach dem Herausnehmen aus dem Wasser gewogen. Diese Masse wird als $m_2$ angegeben.

Anschließend wurden die Probekörper erneut in einen Exsikkator bei 37°C aufbewahrt. Nach 22 Stunden

wurden die Probekörper entnommen, für 2 Stunden in einen zweiten Exsikkator auf 23°C gebracht und dann auf 0,1 mg gewogen. Dieser Zyklus wurde so lange wiederholt, bis eine konstante Masse, $m_3$, erreicht war.

Die Wasseraufnahme, $W_{sp}$, wurde nach folgender Gleichung berechnet:

$$W_{sp} = \frac{m_2 - m_3}{V}$$

Dabei ist

$m_2$ die Masse des Probekörpers nach Wasserlagerung für 7 Tage in $\mu$g;
$m_3$ die Masse des wieder getrockneten Probekörpers in $\mu$g;
V das Volumen des Probekörpers in $mm^3$

Die Bestimmung der Wasseraufnahme erfolgte für die Zahnfüllungsmaterialien aus den Beispielen 2, 3 und 4. Die Ergebnisse sind in der nachfolgenden Tabelle 4 zusammengefasst.

**Tabelle 4**

| | Beispiel 2 | Beispiel 3 | Beispiel 4 |
|---|---|---|---|
| Wasseraufnahme [$\mu g/mm^3$] | 10 | 12 | 15,5 |

## Patentansprüche

1. Zahnfüllungsmaterialien oder Zahnlacke zur Hemmung der Biofilmbildung von *Streptococcus mutans* umfassend:

(a) als Wirksubstanz zur Hemmung der Biofilmbildung von *Streptococcus mutans* eine Menge an Carolacton der Formel (II)

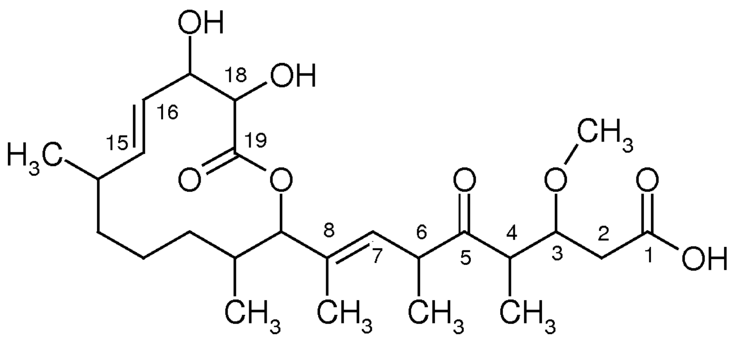

(II)

und
(b) ein Material zur Ausbildung einer Struktur zur Aufnahme und verzögerten Freisetzung der Wirksubstanz, bestehend aus einem, zwei, drei oder mehr polymerisierbaren Monomeren
und
(c) einen oder mehrere partikuläre Füllstoffe
und
(d) ein oder mehrere Additive ausgewählt aus der Gruppe bestehend aus Initiatoren, Beschleuniger und Inhibitoren.

2. Zahnfüllungsmaterial oder Zahnlack nach Anspruch 1, wobei das eine polymerisierbare Monomer bzw. eines, zwei, drei, mehrere oder sämtliche der mehreren polymerisierbaren Monomere der Komponente (b) ausgewählt ist bzw. sind aus der Gruppe der (Meth)acrylate und (Meth)acrylamide, vorzugsweise ausgewählt ist bzw. sind aus der Gruppe bestehend aus Ethylenglykoldimethacrylat (EGDMA), 1,6-Hexandioldimethacrylat (HEDMA), Butandioldimethacrylat, Tetraethylenglykoldimethacrylat, Neopentylglykoldimethacrylat, 2-Hydroxypropyl-1,3-dimethacrylat, 3-Hydroxypropyl-1,2-dimethacrylat, Pentaerythritoldimethacrylat, Triethylenglykoldimethacrylat (TEGDMA), Urethandimethacrylat (7,7,9-Trimethyl-4,13-dioxo-3,14-dioxa-5,12-diazahexadecan-1,16-dioxydimethacrylat (UDMA)), Glycerindimethacrylat (GlyDMA), Bisphenol-A-Glycidyl-Methacrylat (Bis-GMA), 1,12-Dodecandioldimethacrylat (DODMA), Hydroxyethylmethacrylat (HEMA), Polyethylenglykoldimethacrylat (PEGDMA) und ethoxyliertes Bisphenol-A-dimethacrylat (EtoBG).

**3.** Zahnfüllungsmaterial oder Zahnlack nach einem der vorangehenden Ansprüche, umfassend als Wirksubstanz zur Hemmung der Biofilmbildung von *Streptococcus mutans* insgesamt 0,001 μg/mL bis 2500 μg/mL, bevorzugt 0,01 μg/mL bis 250 μg/mL, besonders bevorzugt 0,1 μg/mL bis 25 μg/mL Carolacton der Formel (II), bezogen auf das Volumen des Zahnfüllungsmaterials oder Zahnlacks.

**4.** Zahnfüllungsmaterial oder Zahnlack nach einem der vorangehenden Ansprüche, umfassend in oder als Komponente

(c) einen oder mehrere anorganische partikuläre Füllstoffe, wobei der eine bzw. einer oder mehrere oder sämtliche der anorganischen partikulären Füllstoffe vorzugsweise ausgewählt sind aus der Gruppe bestehend aus silanisierten $SiO_2$-Nanopartikeln, pyrogenen und/oder nicht-agglomerierten und/oder nicht-aggregierten Kieselsäuren, nicht-aggregierten, nicht-agglomerierten $SiO_2$-Nanopartikeln, Dentalglaspartikeln, silanisierten Dentalglaspartikeln, Zirkoniumoxidpartikeln, Aluminiumoxidpartikeln und mittels organischer Strukturelemente oberflächenmodifizierten, anorganischen Füllstoffpartikeln.

**5.** Zahnfüllungsmaterial oder Zahnlack nach einem der vorangehenden Ansprüche, umfassend

(e) ein oder mehrere Additive ausgewählt aus der Gruppe bestehend aus Silber, Silberionen freisetzende Substanzen, Kupfer, Kupferionen freisetzende Substanzen, Zink und Zinkionen freisetzende Substanzen
und/oder
(f) ein, zwei, drei oder mehr Weichmacher, vorzugsweise Polyethylenglycol als Weichmacher
und/oder
(g) sonstige Bestandteile.

**6.** Zahnfüllungsmaterial oder Zahnlack nach einem der vorangehenden Ansprüche, zur Anwendung in einem Verfahren zur therapeutischen Behandlung des menschlichen oder tierischen Körpers,
vorzugsweise zur Anwendung

- in einem Verfahren zum Inhibieren, Reduzieren oder Verhindern von Zahnschäden, insbesondere von Zahnschäden, die durch *Streptococcus mutans* hervorgerufen werden,

und/oder

- in einem Verfahren zum Inhibieren, Reduzieren oder Verhindern von Caries dentium.

**7.** Zahnfüllungsmaterial oder Zahnlack nach einem der vorangehenden Ansprüche, zur Anwendung in einem therapeutischen Verfahren zum Inhibieren, Reduzieren oder Verhindern von Biofilmen, vorzugsweise von bakteriellen Biofilmen.

**8.** Verfahren zum Herstellen eines Zahnfüllungsmaterials oder Zahnlacks wie in einem der Ansprüche 1 bis 7 definiert, mit den folgenden Schritten:

(i) Bereitstellen oder Herstellen (a) einer Menge an Carolacton der Formel (II), als Wirksubstanz zur Hemmung der Biofilmbildung von *Streptococcus mutans,* vorzugsweise als alkoholische Lösung,
(ii) Bereitstellen oder Herstellen (b) eines Materials zur Ausbildung einer Struktur zur Aufnahme und verzögerten Freisetzung der Wirksubstanz bestehend aus ein, zwei, drei oder mehr polymerisierbaren Monomeren,
(iii) Bereitstellen oder Herstellen (c) eines oder mehrerer partikulärer Füllstoffe
(iv) Bereitstellen oder Herstellen (d) eines oder mehrerer Additive ausgewählt aus der Gruppe bestehend aus Initiatoren, Beschleuniger und Inhibitoren,
(v) optional Bereitstellen oder Herstellen

(e) eines oder mehrerer Additive ausgewählt aus der Gruppe bestehend aus Silber, Silberionen freisetzende Substanzen, Kupfer, Kupferionen freisetzende Substanzen, Zink und Zinkionen freisetzende Substanzen,
(f) von ein, zwei, drei oder mehr Weichmachern, vorzugsweise von Polyethylenglycol als Weichmacher

und/oder
(g) von ein oder mehr sonstigen Bestandteilen

und
(vi) Vermischen der bereitgestellten und/oder hergestellten Komponenten.

**9.** Kit umfassend:

- ein Zahnfüllungsmaterial oder einen Zahnlack nach einem der Ansprüche 1 bis 7 und
- ein oder mehrere weitere Bestandteile ausgewählt aus der Gruppe bestehend aus Haftvermittler, Abutments, Implantate, Formwerkzeuge, Abformmaterialien, Farbskalen, Farbmustern und Lichthärtegeräten.

**10.** Verwendung von Carolacton der Formel (II) oder einer Mischung umfassend Carolacton der Formel (II) zur Herstellung eines Zahnfüllungsmaterials oder Zahnlacks nach einem der Ansprüche 1 bis 7.

**11.** Verfahren zur Herstellung eines die Biofilmbildung hemmenden dentalen Bauteils, mit folgenden Schritten:

- Bereitstellen eines dentalen Bauteils,
- Bereitstellen oder Herstellen eines Zahnfüllungsmaterials oder Zahnlacks wie in einem der Ansprüche 1 bis 7 definiert,
- Applizieren des bereitgestellten bzw. hergestellten Zahnfüllungsmaterials oder Zahnlacks auf das dentale Bauteil,
- optional Formen des applizierten Zahnfüllungsmaterials oder Zahnlacks auf dem dentalen Bauteil,
- optional Aushärten oder härten lassen des applizierten, optional des geformten Zahnfüllungsmaterials oder Zahnlacks auf dem dentalen Bauteil und
- optional Nachbehandeln des ausgehärteten Zahnfüllungsmaterials oder Zahnlacks auf dem dentalen Bauteil, vorzugsweise durch Polieren und/oder Schleifen.

**12.** Verfahren zur Herstellung eines die Biofilmbildung hemmenden dentalen Bauteils, mit folgenden Schritten:

- Bereitstellen oder Herstellen eines Zahnfüllungsmaterials wie in einem der Ansprüche 1 bis 7 definiert,
- Formen des Zahnfüllungsmaterials,
- Aushärten oder härten lassen des geformten Zahnfüllungsmaterials und
- optional Nachbehandeln des ausgehärteten geformten Zahnfüllungsmaterials.

## Claims

**1.** Tooth filling materials or dental varnishes for inhibiting the formation of biofilms of *Streptococcus mutans* comprising:

(a) a quantity of carolactone of the formula (II) as the active substance for inhibiting the formation of biofilms of *Streptococcus mutans*

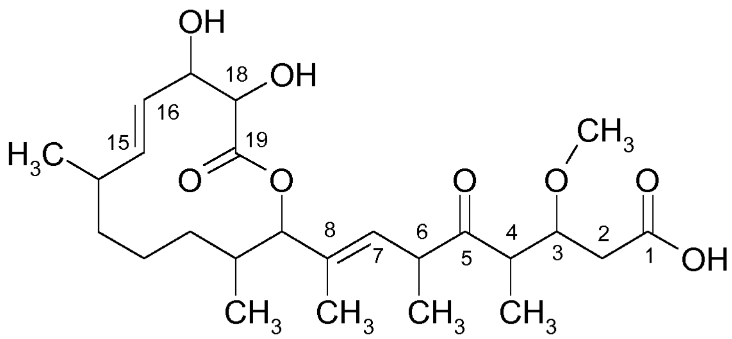

(II)

and
(b) a material for forming a structure for the uptake and delayed release of the active substance consisting of

one, two, three or more polymerisable monomers,
and
(c) one or more particulate fillers
and
(d) one or more additives selected from the group consisting of initiators, accelerators and inhibitors.

2. Tooth filling material or dental varnish according to claim 1, wherein the one polymerisable monomer or one, two, three, a plurality of or all the plurality of polymerisable monomers of the component (b) is or are selected from the group of (meth)acrylates and (meth)acrylamides, is or are preferably selected from the group consisting of ethylene glycol dimethacrylate (EGDMA), 1,6-hexanediol dimethacrylate (HEDMA), butanediol dimethacrylate, tetraethylene glycol dimethacrylate, neopentyl glycol dimethacrylate, 2-hydroxypropyl-1,3-dimethacrylate, 3-hydroxypropyl-1,2-dimethacrylate, pentaerythritol dimethacrylate, triethylene glycol dimethacrylate (TEGDMA), urethane dimethacrylate (7,7,9-trimethyl-4,13-dioxo-3,14-dioxa-5,12-diazahexadecan-1,16-dioxydimethacrylate (UDMA)), glycerol dimethacrylate (GlyDMA), bisphenol-A-glycidyl-methacrylate (Bis-GMA), 1,12-dodecandiol dimethacrylate (DODMA), hydroxyethyl methacrylate (HEMA), polyethylene glycol dimethacrylate (PEGDMA) and ethoxylated bisphenol-A-dimethacrylate (EtoBG).

3. Tooth filling material or dental varnish according to any one of the preceding claims, comprising as the active substance for inhibiting the biofilm formation of *Streptococcus mutans* a total of from 0.001 μg/mL to 2500 μg/mL, preferably from 0.01 μg/mL to 250 μg/mL, particularly preferably from 0.1 μg/mL to 25 μg/mL of carolactone of the formula (II), in relation to the volume of the tooth filling material or dental varnish.

4. Tooth filling material or dental varnish according to any one of the preceding claims, comprising in or as component

(c) one or more inorganic particulate fillers, wherein the one or one or more or all of the inorganic particulate filling materials preferably being selected from the group consisting of silanised $SiO_2$ nanoparticles, pyrogenic and/or non-agglomerated and/or non-aggregated silicic acids, non-aggregated, non-agglomerated $SiO_2$ nanoparticles, dental glass particles, silanised dental glass particles, zirconium oxide particles, aluminium oxide particles and inorganic filling material particles surface-modified by means of organic structural elements.

5. Tooth filling material or dental varnish according to any one of the preceding claims, comprising

(e) one or more additives selected from the group consisting of silver, substances releasing silver ions, copper, substances releasing copper ions, zinc and substances releasing zinc ions,
and/or
(f) one, two, three or more softeners, preferably polyethylene glycol as the softener, and/or
(g) other constituents.

6. Tooth filling material or dental varnish according to any one of the preceding claims, for use in a method for the therapeutic treatment of the human or animal body,
preferably for use

- in a method for inhibiting, reducing or preventing tooth damage, in particular tooth damage which is caused by *Streptococcus mutans,*

and/or

- in a method for inhibiting, reducing or preventing dental caries.

7. Tooth filling material or dental varnish according to any one of the preceding claims, for use in a therapeutic method for inhibiting, reducing or preventing biofilms, preferably bacterial biofilms.

8. Method for producing a tooth filling material or dental varnish as defined in any one of claims 1 to 7, having the following steps:

(i) providing or producing (a) a quantity of carolactone of the formula (II), as the active substance for inhibiting the biofilm formation of *Streptococcus mutans,* preferably as an alcoholic solution,
(ii) providing or producing (b) a material for forming a structure for the uptake and delayed release of the active

substance consisting of one, two, three or more polymerisable monomers,
(iii) providing or producing (c) one or more particulate fillers,
(iv) providing or producing (d) one or more additives selected from the group consisting of initiators, accelerators and inhibitors,
(v) optionally providing or producing

(e) one or more additives selected from the group consisting of silver, substances releasing silver ions, copper, substances releasing copper ions, zinc and substances releasing zinc ions,
(f) one, two, three or more softeners, preferably polyethylene glycol as the softener
and/or
(g) one or more other constituents

and
(vi) mixing the components provided and/or produced.

**9.** Kit comprising:

- a tooth filling material or dental varnish according to any one of claims 1 to 7 and
- one or more additional constituents selected from the group consisting of adhesives, abutments, implants, forming tools, impression materials, colour scales, colour guides and light hardening devices.

**10.** Use of carolactone of the formula (II), or an admixture comprising carolactone of the formula (II), for producing a tooth filling material or dental varnish according to any one of claims 1 to 7.

**11.** Method for producing a dental component which inhibits the biofilm formation having the following steps:

- providing a dental component,
- providing or producing a tooth filling material or dental varnish as defined in any one of claims 1 to 7,
- applying the provided or produced tooth filling material or dental varnish to the dental component,
- optionally forming the applied tooth filling material or dental varnish on the dental component,
- optionally hardening, or allowing to harden of the applied tooth filling material or dental varnish, which is optionally formed, on the dental component and
- optionally further post-treating the hardened tooth filling material or dental varnish on the dental component, preferably by polishing and/or grinding.

**12.** Method for producing a dental component which inhibits the biofilm formation having the following steps:

- providing or producing a tooth filling material as defined in any one of claims 1 to 7,
- forming the tooth filling material,
- hardening, or allowing to harden of the formed tooth filling material and
- optionally further post-treating the hardened formed tooth filling material.

## Revendications

**1.** Matériaux d’obturation dentaire ou laques dentaires destinés à empêcher la formation d’un biofilm de *Streptococcus mutans,* comprenant :

(a) comme substance active destinée à empêcher la formation d’un biofilm de *Streptococcus mutans,* une quantité de carolactone de formule (II)

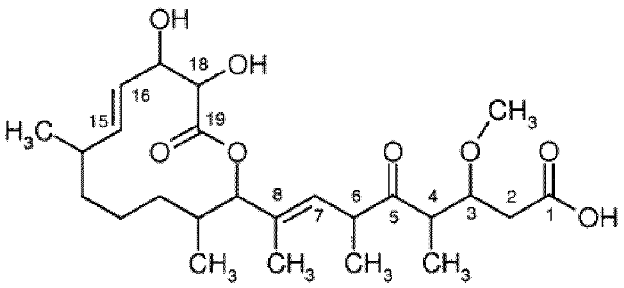

(II)

et

(b) un matériau destiné à former une structure pour la réception et la libération retardée de la substance active, constituée d'un, de deux, de trois monomères polymérisables ou plus,

et

(c) une ou plusieurs charges particulaires

et

(d) un ou plusieurs additifs choisis dans le groupe constitué des initiateurs, des accélérateurs et des inhibiteurs.

**2.** Matériau d'obturation dentaire ou laque dentaire selon la revendication 1, dans lequel le monomère polymérisable ou un, deux, trois, plusieurs ou la totalité des plusieurs monomères polymérisables du composant (b) sont choisis dans le groupe constitué des (méth)acrylates et des (méth)acrylamides, de préférence choisis dans le groupe constitué du diméthacrylate d'éthylène glycol (EGDMA), du diméthacrylate de 1,6-hexanediol (HEDMA), du diméthacrylate de butanediol, du diméthacrylate de tétraéthylène glycol, du diméthacrylate de néopentylglycol, du 1,3-diméthacrylate de 2-hydroxypropyle, du 1,2-diméthacrylate de 3-hydroxypropyle, du diméthacrylate de pentaérythritol, du diméthacrylate de triéthylène glycol (TEGDMA), du diméthacrylate d'uréthane (7,7,9-triméthyl-4,13-dioxo-3,14-dioxa-5,12-diazahexanedécane-1,16-dioxy-diméthacrylate (UDMA)), du diméthacrylate de glycérine (GlyDMA), du méthacrylate de bisphénol-A-glycidyle (Bis-GMA), du diméthacrylate de 1,12-dodécanediol (DODMA), du méthacrylate d'hydroxyéthyle (HEMA), du diméthacrylate de polyéthylène glycol (PEGDMA) et du diméthacrylate de bisphénol-A éthoxylé (EtoBG).

**3.** Matériau d'obturation dentaire ou laque dentaire selon l'une des revendications précédentes, comprenant, comme substance active destinée à empêcher la formation d'un biofilm de *Streptococcus mutans,* au total 0,001 $\mu$g/ml à 2500 $\mu$g/ml, de préférence 0,01 $\mu$g/ml à 250 $\mu$g/ml, de manière particulièrement préférée 0,1 $\mu$g/ml à 25 $\mu$g/ml de carolactone de formule (II), par rapport au volume du matériau d'obturation dentaire ou de laque dentaire.

**4.** Matériau d'obturation dentaire ou laque dentaire selon l'une des revendications précédentes, comprenant dans un composant ou comme composant

(c) une ou plusieurs charges particulaires inorganiques, dans lequel l'une, ou plusieurs, ou la totalité des charges particulaires inorganiques sont choisies de préférence dans le groupe constitué des nanoparticules de $SiO_2$ silanisées, des acides siliciques pyrogènes et/ou non agglomérés et/ou non agrégés, des nanoparticules de $SiO_2$ non agglomérées, non agrégées, des particules de verre dentaire, des particules de verre dentaire silanisées, des particules d'oxyde de zirconium, des particules d'oxyde d'aluminium et des particules de charges inorganiques, modifiées en surface au moyen d'éléments structurels organiques.

**5.** Matériau d'obturation dentaire ou laque dentaire selon l'une des revendications précédentes, comprenant

(e) un ou plusieurs additifs choisis dans le groupe constitué de l'argent, des substances libérant des ions argent, du cuivre, des substances libérant des ions cuivre, du zinc et des substances libérant des ions zinc

et/ou

(f) un, deux, trois plastifiants ou plus, de préférence le polyéthylène glycol comme plastifiant et/ou

(g) d'autres composants.

**6.** Matériau d'obturation dentaire ou laque dentaire selon l'une des revendications précédentes, pour une application dans un procédé destiné au traitement thérapeutique du corps humain ou animal,
de préférence pour l'application

- dans un procédé destiné à inhiber, réduire ou empêcher les lésions dentaires, en particulier les lésions dentaires provoquées par *Streptococcus mutans,*

et/ou

- dans un procédé destiné à inhiber, réduire ou empêcher les caries dentaires.

**7.** Matériau d'obturation dentaire ou laque dentaire selon l'une des revendications précédentes, pour une application dans un procédé thérapeutique destiné à inhiber, réduire ou empêcher les biofilms, de préférence les biofilms bactériens.

**8.** Procédé de fabrication d'un matériau d'obturation dentaire ou d'une laque dentaire, tels que définis dans l'une des revendications 1 à 7, avec les étapes suivantes consistant à :

(i) mettre à disposition ou fabriquer (a) une quantité de carolactone de formule (II), comme substance active destinée à empêcher la formation d'un biofilm de *Streptococcus mutans,* de préférence sous forme de solution alcoolique,
(ii) mettre à disposition ou fabriquer (b) un matériau pour la formation d'une structure pour la réception et la libération retardée de la substance active constituée d'un, de deux, de trois monomères polymérisables ou plus,
(iii) mettre à disposition ou fabriquer (c) une ou plusieurs charges particulaires
(iv) mettre à disposition ou fabriquer (d) un ou plusieurs additifs choisis dans le groupe constitué des initiateurs, des accélérateurs et des inhibiteurs,
(v) mettre à disposition ou fabriquer éventuellement

(e) un ou plusieurs additifs choisis dans le groupe constitué de l'argent, des substances libérant des ions argent, du cuivre, des substances libérant des ions cuivre, du zinc et des substances libérant des ions zinc,
(f) un, deux, trois plastifiants ou plus, de préférence du polyéthylène glycol comme plastifiant
et/ou
(g) un ou plusieurs autres composants

et
(vi) mélanger des composants mis à disposition et/ou fabriqués.

**9.** Kit comprenant :

- un matériau d'obturation dentaire ou une laque dentaire selon l'une des revendications 1 à 7, et
- un ou plusieurs autres composants choisis dans le groupe constitué des agents d'adhérence, des piliers, des implants, des outils de formage, des matériaux de façonnage, des échelles de couleur, des modèles de couleur et des appareils photodurcisseurs.

**10.** Utilisation de la carolactone de formule (II) ou d'un mélange comprenant de la carolactone de formule (II) pour la fabrication d'un matériau d'obturation dentaire ou d'une laque dentaire selon l'une des revendications 1 à 7.

**11.** Procédé pour la fabrication d'un composant dentaire inhibant la formation d'un biofilm, avec les étapes consistant à :

- mettre à disposition un composant dentaire,
- mettre à disposition ou fabriquer un matériau d'obturation dentaire ou une laque dentaire, tels que définis dans l'une des revendications 1 à 7,
- appliquer le matériau d'obturation dentaire ou la laque dentaire mis à disposition ou fabriqué sur le composant dentaire,
- former éventuellement le matériau d'obturation dentaire ou la laque dentaire appliqués sur le composant dentaire,
- durcir éventuellement ou laisser durcir le matériau d'obturation dentaire ou la laque dentaire appliqués, éventuellement formés, sur le composant dentaire, et
- retraiter éventuellement le matériau d'obturation ou la laque dentaire durcis sur le composant dentaire, de préférence par polissage et/ou meulage.

**12.** Procédé de fabrication d'un composant dentaire inhibant la formation d'un biofilm, avec les étapes suivantes consistant à :

- mettre à disposition ou fabriquer un matériau d’obturation dentaire tel que défini dans l’une des revendications 1 à 7,
- former le matériau d’obturation dentaire,
- durcir ou laisser durcir le matériau d’obturation dentaire formé et
- retraiter éventuellement le matériau d’obturation dentaire formé durci.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- GB 1433303 A
- DE 102009035970
- JP 10025218 A
- DE 102005042078 A1
- DE 102009035970 A1
- US 20120095114 A1
- US 6207139 B
- US 20120171129 A1
- WO 2004078154 A
- WO 2009030773A10 A
- DE 3941629 A1
- DE 2405578 A1
- US 20020065337 A
- DE 19508586 A1
- WO 0069392 A
- US 6387981 B
- DE 102006019092 A1
- DE 3941629 C2
- DE 102006019092
- DE 60116142
- DE 3801511 C2
- DE 102006050153 A1
- EP 0184095 B1
- DE 4231579 C2
- EP 0366977 B1
- US 7081485 B2
- DE 3236026 A1
- US 20070027229 A1
- EP 0262629 B1
- EP 0073413 A
- US 7148382 B2
- US 5761169 A
- DE 19708294 A1
- EP 0057474 A
- EP 0047902 A
- EP 0007508 A
- DE 60029481 T2
- EP 0980682 B1
- EP 0948955 B1
- EP 1236459 B1
- EP 0173567 A2
- US 4772530 A
- US 4954414 A
- US 4874450 A
- US 5055372 A
- US 5057393 A
- EP 1720506 A
- EP 1839640 A
- DE 1495520
- WO 02092021 A
- WO 02092023 A
- EP 0059451 A
- EP 0783880 B1
- DE 10119831 A1
- EP 1563821 A1


### In der Beschreibung aufgeführte Nicht-Patentliteratur


- **J.-P. FOUASSIER.** Photoinitiation, Photopolymerization and Photocuring. Hanser Publishers, 1995
- Radiation Curing in Polymer Science and Technology. Elsevier Applied Science, 1993, vol. II